# EUROPEAN PATENT SPECIFICATION

(11) **EP 2 707 720 B1**
(45) Date of publication and mention of the grant of the patent: **14.03.2018**
(21) Application number: 12781922.5
(22) Date of filing: 10.05.2012
(51) Int. Cl.: G01N 33/574

(54) **DIAGNOSIS AND PROGNOSIS OF TRIPLE NEGATIVE BREAST AND OVARIAN CANCER**
DIAGNOSE UND PROGNOSE VON DREIFACH NEGATIVEM BRUST- UND OVARIALKREBS
DIAGNOSTIC ET PRONOSTIC DE CANCER DU SEIN TRIPLE-NÉGATIF ET DE L'OVAIRE

(30) Priority: 11.05.2011 US 201161485043 P
(43) Date of publication of application: 19.03.2014
(73) Proprietor: Alper Biotech, LLC, Rockville, Maryland 20850 (US)
(72) Inventor: ALPER, Ozge, Bethesda,MD 20814 (US)
(74) Representative: Richards, William John
(86) International application number: PCT/US2012/037327
(87) International publication number: WO 2012/154957

(56) References cited:
- EP-A1- 2 499 160
- US-A1- 2008 182 246
- US-A1- 2010 210 738
- US-B2- 7 705 120
- KORKOLA JAMES E ET AL: "Identification of a robust gene signature that predicts breast cancer outcome in independent data sets", BMC CANCER, BIOMED CENTRAL, LONDON, GB, vol. 7, no. 1, 11 April 2007 (2007-04-11), page 61, XP021023142, ISSN: 1471-2407, DOI: 10.1186/1471-2407-7-61
- J. HANNEMANN: "Changes in Gene Expression Associated With Response to Neoadjuvant Chemotherapy in Breast Cancer", JOURNAL OF CLINICAL ONCOLOGY, vol. 23, no. 15, 20 May 2005 (2005-05-20), pages 3331-3342, XP55149530, ISSN: 0732-183X, DOI: 10.1200/JCO.2005.09.077
- SPEERS, C. NOVEL STRATEGIES FOR THE TREATMENT OF ESTROGEN RECEPTOR-NEGATIVE BREAST CANCER. April 2009, page 300 PP, XP003030260
- CHOI ET AL.: 'Triple-negative, basal-like, and quintuple-negative breast cancers: better prediction model for survival.' BMC CANCER vol. 10, 2010, pages 507 - 522, XP021075329
- LI ET AL.: 'Identification of glia maturation factor beta as an independent prognostic predictor for serous ovarian cancer.' EUROPEAN JOURNAL OF CANCER vol. 46, no. 11, 2010, pages 2104 - 2118, XP027113599
- WANG ET AL.: 'Gene-expression profiles to predict distant metastasis of lymph-node-negative primary breast cancer.' THE LANCET vol. 365, 2005, pages 671 - 679, XP002713581

### SEQUENCE LISTING

<110> ALPER, OZGE
<120> DIAGNOSIS AND PROGNOSIS OF TRIPLE NEGATIVE BREAST AND OVARIAN CANCER
<130> 23440.018
<160> 52
<170> PatentIn version 3.5
<210> 1
   <211> 115
   <212> PRT
   <213> Artificial Sequence
<220>
   <223> Description of Artificial Sequence: Synthetic polypeptide
<400> 1
   Val Gln Leu Gln Gln Ser Gly Pro Glu Leu Val Lys Pro Gly Ala Ser
   1               5                   10                  15
   Val Lys Met Ser Cys Lys Ala Ser Gly Tyr Thr Phe Thr Ser Tyr Val
               20                  25                  30
   Met His Trp Val Lys Gln Lys Pro Gly Gln Gly Leu Glu Trp Ile Gly
           35                  40                  45
   Tyr Ile Asn Pro Tyr Asn Glu Gly Thr Lys Tyr Asn Glu Lys Phe Lys
       50                  55                  60
   Gly Lys Ala Thr Leu Thr Ser Asp Lys Ser Ser Ser Thr Ala Tyr Met
   65                  70                  75                  80
   Glu Leu Ser Ser Leu Thr Ser Glu Asp Ser Ala Val Tyr Tyr Cys Ala
                   85                  90                  95
   Arg Ser Thr Met Ile Thr Thr Gly Phe Ala Tyr Trp Gly Gln Gly Thr
               100                 105                 110
   Thr Val Thr
           115
<210> 2
   <211> 9
   <212> PRT
   <213> Artificial Sequence
<220>
   <223> Description of Artificial Sequence: Synthetic peptide
<400> 2
   Tyr Thr Phe Thr Ser Tyr Val Met His
   1               5
<210> 3
   <211> 17
   <212> PRT
   <213> Artificial Sequence
<220>
   <223> Description of Artificial Sequence: Synthetic peptide
<400> 3
   Tyr Ile Asn Pro Tyr Asn Glu Gly Thr Lys Tyr Asn Glu Lys Phe Lys
   1               5                   10                  15
   Gly
<210> 4
   <211> 10
   <212> PRT
   <213> Artificial Sequence
<220>
   <223> Description of Artificial Sequence: Synthetic peptide
<400> 4
   Ser Thr Met Ile Thr Thr Gly Phe Ala Tyr
   1               5                   10
<210> 5
   <211> 106
   <212> PRT
   <213> Artificial Sequence
<220>
   <223> Description of Artificial Sequence: Synthetic polypeptide
<400> 5
   Asp Ile Leu Met Thr Gln Ser Pro Ser Ser Leu Ser Ala Ser Leu Gly
   1               5                   10                  15
   Gly Lys Val Thr Ile Thr Cys Lys Ala Ser Gln Asp Ile Asn Lys Tyr
               20                  25                  30
   Ile Ala Trp Tyr Gln His Lys Pro Gly Glu Gly Pro Arg Leu Leu Ile
           35                  40                  45
   His Tyr Thr Ser Thr Leu Gln Pro Gly Ile Pro Ser Arg Phe Ser Gly
       50                  55                  60
   Ser Gly Ser Gly Arg Asp Tyr Ser Phe Ser Ile Thr Asn Leu Glu Pro
   65                  70                  75                  80
   Glu Asp Ile Ala Thr Tyr Tyr Cys Leu Gln Tyr Asp Asn Leu Trp Thr
                   85                  90                  95
   Phe Gly Gly Gly Thr Lys Leu Glu Ile Lys
               100                 105
<210> 6
   <211> 11
   <212> PRT
   <213> Artificial Sequence
<220>
   <223> Description of Artificial Sequence: Synthetic peptide
<400> 6
   Lys Ala Ser Gln Asp Ile Asn Lys Tyr Ile Ala
   1 5 10
<210> 7
   <211> 7
   <212> PRT
   <213> Artificial Sequence
<220>
   <223> Description of Artificial Sequence: Synthetic peptide
<400> 7
   Tyr Thr Ser Thr Leu Gln Pro
   1 5
<210> 8
   <211> 9
   <212> PRT
   <213> Artificial Sequence
<220>
   <223> Description of Artificial Sequence: Synthetic peptide
<400> 8
   Cys Leu Gln Tyr Asp Asn Leu Trp Thr
   1 5
<210> 9
   <211> 142
   <212> PRT
   <213> Artificial Sequence
<220>
   <223> Description of Artificial Sequence: Synthetic polypeptide
<400> 9
   Met Ser Glu Ser Leu Val Val Cys Asp Val Ala Glu Asp Leu Val Glu
   1               5                   10                  15
   Lys Leu Arg Lys Phe Arg Phe Arg Lys Glu Thr Asn Asn Ala Ala Ile
               20                  25                  30
   Ile Met Lys Ile Asp Lys Asp Lys Arg Leu Val Val Leu Asp Glu Glu
           35                  40                  45
   Leu Glu Gly Ile Ser Pro Asp Glu Leu Lys Asp Glu Leu Pro Glu Arg
       50                  55                  60
   Gln Pro Arg Phe Ile Val Tyr Ser Tyr Lys Tyr Gln His Asp Asp Gly
   65                  70                  75                  80
   Arg Val Ser Tyr Pro Leu Cys Phe Ile Phe Ser Ser Pro Val Gly Cys
                   85                  90                  95
   Lys Pro Glu Gln Gln Met Met Tyr Ala Gly Ser Lys Asn Lys Leu Val
               100                 105                 110
   Gln Thr Ala Glu Leu Thr Lys Val Phe Glu Ile Arg Asn Thr Glu Asp
           115                 120                 125
   Leu Thr Glu Glu Trp Leu Arg Glu Lys Leu Gly Phe Phe His
       130                 135                 140
<210> 10
   <211> 141
   <212> PRT
   <213> Artificial Sequence
<220>
   <223> Description of Artificial Sequence: Synthetic polypeptide
<400> 10
   Ser Glu Ser Leu Val Val Cys Asp Val Ala Glu Asp Leu Val Glu Lys
   1               5                   10                  15
   Leu Arg Lys Phe Arg Phe Arg Lys Glu Thr Asn Asn Ala Ala Ile Ile
               20                  25                  30
   Met Lys Ile Asp Lys Asp Lys Arg Leu Val Val Leu Asp Glu Glu Leu
           35                  40                  45
   Glu Gly Ile Ser Pro Asp Glu Leu Lys Asp Glu Leu Pro Glu Arg Gln
       50                  55                  60
   Pro Arg Phe Ile Val Tyr Ser Tyr Lys Tyr Gln His Asp Asp Gly Arg
   65                  70                  75                  80
   Val Ser Tyr Pro Leu Cys Phe Ile Phe Ser Ser Pro Val Gly Cys Lys
                   85                  90                  95
   Pro Glu Gln Gln Met Met Tyr Ala Gly Ser Lys Asn Lys Leu Val Gln
               100                 105                 110
   Thr Ala Glu Leu Thr Lys Val Phe Glu Ile Arg Asn Thr Glu Asp Leu
           115                 120                 125
   Thr Glu Glu Trp Leu Arg Glu Lys Leu Gly Phe Phe His
       130                 135                 140
<210> 11
   <211> 140
   <212> PRT
   <213> Artificial Sequence
<220>
   <223> Description of Artificial Sequence: Synthetic polypeptide
<400> 11
   Met Ser Glu Ser Leu Val Val Cys Asp Val Ala Glu Asp Leu Val Glu
   1               5                   10                  15
   Lys Leu Arg Lys Phe Arg Phe Arg Lys Glu Thr Asn Asn Ala Ala Ile
               20                  25                  30
   Ile Met Lys Ile Asp Lys Asp Lys Arg Leu Val Val Leu Asp Glu Glu
           35                  40                  45
   Leu Glu Gly Ile Ser Pro Asp Glu Leu Lys Asp Glu Leu Pro Glu Arg
       50                  55                  60
   Gln Pro Arg Phe Ile Val Tyr Ser Tyr Lys Tyr Gln His Asp Asp Gly
   65                  70                  75                  80
   Arg Val Ser Tyr Pro Leu Cys Phe Ile Phe Ser Ser Pro Val Gly Cys
                   85                  90                  95
   Lys Pro Glu Gln Gln Met Met Tyr Ala Gly Ser Lys Asn Lys Leu Val
               100                 105                 110
   Gln Thr Ala Glu Leu Thr Lys Val Phe Glu Ile Arg Asn Thr Glu Asp
           115                 120                 125
   Leu Thr Glu Glu Trp Leu Arg Glu Lys Leu Gly Phe
   130 135 140
<210> 12
   <211> 139
   <212> PRT
   <213> Artificial Sequence
<220>
   <223> Description of Artificial Sequence: Synthetic polypeptide
<400> 12
   Ser Glu Ser Leu Val Val Cys Asp Val Ala Glu Asp Leu Val Glu Lys
   1 5 10 15
   Leu Arg Lys Phe Arg Phe Arg Lys Glu Thr Asn Asn Ala Ala Ile Ile
   20 25 30
   Met Lys Ile Asp Lys Asp Lys Arg Leu Val Val Leu Asp Glu Glu Leu
   35 40 45
   Glu Gly Ile Ser Pro Asp Glu Leu Lys Asp Glu Leu Pro Glu Arg Gln
   50 55 60
   Pro Arg Phe Ile Val Tyr Ser Tyr Lys Tyr Gln His Asp Asp Gly Arg
   65 70 75 80
   Val Ser Tyr Pro Leu Cys Phe Ile Phe Ser Ser Pro Val Gly Cys Lys
   85 90 95
   Pro Glu Gln Gln Met Met Tyr Ala Gly Ser Lys Asn Lys Leu Val Gln
   100 105 110
   Thr Ala Glu Leu Thr Lys Val Phe Glu Ile Arg Asn Thr Glu Asp Leu
   115 120 125
   Thr Glu Glu Trp Leu Arg Glu Lys Leu Gly Phe
   130 135
<210> 13
   <211> 400
   <212> **DNA**
   <213> Artificial Sequence
<220>
   <223> Description of Artificial Sequence: Synthetic polynucleotide
<220>
   <221> modified_base
   <222> (1)..(12)
   <223> a, c, t, g, unknown or other
<220>
   <221> modified_base
   <222> (16)..(16)
   <223> a, c, t, g, unknown or other
<400> 13
   nnnnnnnnnn nngcgnttcg ccctttgagg tgcggaggag tccactctga ggtccagctg      60
   cagcagtctg gacctgagct ggtaaagcct ggggcttcag tgaagatgtc ctgcaaggct     120
   tctggataca cattcactag ctatgttatg cactgggtga agcagaagcc tgggcagggc     180
   cttgagtgga ttggatatat taatccttac aatgaaggaa ctaagtacaa tgagaagttc     240
   aaaggcaagg ccacactgac ttcagacaaa tcctccagca cagcctacat ggagctcagc     300
   agcctgacct ctgaggactc tgcggtctat tattgtgcaa gatcgactat gattacgacg     360
   gggtttgctt actggggcca agggaccacg gtcacaaggg                          400
<210> 14
   <211> 400
   <212> DNA
   <213> Artificial Sequence
<220>
   <223> Description of Artificial Sequence: Synthetic polynucleotide
<220>
   <221> modified_base
   <222> (1)..(10)
   <223> a, c, t, g, unknown or other
<220>
   <221> modified_base
   <222> (12)..(14)
   <223> a, c, t, g, unknown or other
<400> 14
   nnnnnnnnnn gnnnacgatt cgcccttgac attctgatga cccagtctcc atcctcactg      60
   tctgcatctc tgggaggcaa agtcaccatc acttgcaagg caagccaaga cattaacaag     120
   tatatagctt ggtaccaaca caagcctgga gaaggtccta ggctgctcat acattacaca     180
   tctacattac agccaggcat cccatcaagg ttcagtggaa gtgggtctgg gagagattat     240
   tccttcagca tcaccaacct ggaacctgaa gatattgcaa cttattattg tctacagtat     300
   gataatctgt ggacgttcgg tggaggcacc aagctggaaa tcaaacgggc tgatgctgcc     360
   caactgtatc catcttccca agggcgaatt cgcggccgct                          400
<210> 15
   <211> 11
   <212> PRT
   <213> Artificial Sequence
<220>
   <223> Description of Artificial Sequence: Synthetic peptide
<400> 15
   Lys Leu Val Gln Thr Ala Glu Leu Thr Lys Val
   1               5                   10
<210> 16
   <211> 12
   <212> PRT
   <213> Artificial Sequence
<220>
   <223> Description of Artificial Sequence: Synthetic peptide
<400> 16
   Lys Glu Thr Asn Asn Ala Ala Ile Ile Met Lys Ile
   1               5                   10
<210> 17
   <211> 13
   <212> PRT
   <213> Artificial Sequence
<220>
   <223> Description of Artificial Sequence: Synthetic peptide
<400> 17
   Arg Asn Thr Glu Asp Leu Thr Glu Glu Trp Leu Arg Glu
   1               5                   10
<210> 18
   <211> 18
   <212> PRT
   <213> Artificial Sequence
<220>
   <223> Description of Artificial Sequence: Synthetic peptide
<400> 18
   Ser Thr Met Ile Thr Thr Gly Phe Ala Tyr Trp Gly Gln Gly Thr Thr
   1               5                   10                  15
   Val Thr
<210> 19
   <211> 12
   <212> PRT
   <213> Artificial Sequence
<220>
   <223> Description of Artificial Sequence: Synthetic peptide
<400> 19
   Trp Thr Phe Gly Gly Gly Thr Lys Leu Glu Ile Lys
   1               5                   10
<210> 20
   <211> 347
   <212> DNA
   <213> Artificial Sequence
<220>
   <223> Description of Artificial Sequence: Synthetic polynucleotide
<220>
   <221> CDS
   <222> (4)..(347)
<400> 20
   gag gtc cag ctg cag cag tct gga cct gag ctg gta aag cct ggg gct        48
       Val Gln Leu Gln Gln Ser Gly Pro Glu Leu Val Lys Pro Gly Ala
       1               5                   10                  15
   tca gtg aag atg tcc tgc aag gct tct gga tac aca ttc act agc tat        96
   Ser Val Lys Met Ser Cys Lys Ala Ser Gly Tyr Thr Phe Thr Ser Tyr
                   20                  25                  30
   gtt atg cac tgg gtg aag cag aag cct ggg cag ggc ctt gag tgg att       144
   Val Met His Trp Val Lys Gln Lys Pro Gly Gln Gly Leu Glu Trp Ile
               35                  40                  45
   gga tat att aat cct tac aat gaa gga act aag tac aat gag aag ttc       192
   Gly Tyr Ile Asn Pro Tyr Asn Glu Gly Thr Lys Tyr Asn Glu Lys Phe
           50                  55                  60
   aaa ggc aag gcc aca ctg act tca gac aaa tcc tcc agc aca gcc tac       240
   Lys Gly Lys Ala Thr Leu Thr Ser Asp Lys Ser Ser Ser Thr Ala Tyr
       65                  70                  75
   atg gag ctc agc agc ctg acc tct gag gac tct gcg gtc tat tat tgt       288
   Met Glu Leu Ser Ser Leu Thr Ser Glu Asp Ser Ala Val Tyr Tyr Cys
   80                  85                  90                  95
   gca aga tcg act atg att acg acg ggg ttt gct tac tgg ggc caa ggg       336
   Ala Arg Ser Thr Met Ile Thr Thr Gly Phe Ala Tyr Trp Gly Gln Gly
                   100                 105                 110
   acc acg gtc ac                                                        347
   Thr Thr Val Thr
               115
<210> 21
   <211> 115
   <212> PRT
   <213> Artificial Sequence
<220>
   <223> Description of Artificial Sequence: Synthetic polypeptide
<400> 21
   Val Gln Leu Gln Gln Ser Gly Pro Glu Leu Val Lys Pro Gly Ala Ser
   1               5                   10                  15
   Val Lys Met Ser Cys Lys Ala Ser Gly Tyr Thr Phe Thr Ser Tyr Val
               20                  25                  30
   Met His Trp Val Lys Gln Lys Pro Gly Gln Gly Leu Glu Trp Ile Gly
           35                  40                  45
   Tyr Ile Asn Pro Tyr Asn Glu Gly Thr Lys Tyr Asn Glu Lys Phe Lys
       50                  55                  60
   Gly Lys Ala Thr Leu Thr Ser Asp Lys Ser Ser Ser Thr Ala Tyr Met
   65                  70                  75                  80
   Glu Leu Ser Ser Leu Thr Ser Glu Asp Ser Ala Val Tyr Tyr Cys Ala
                   85                  90                  95
   Arg Ser Thr Met Ile Thr Thr Gly Phe Ala Tyr Trp Gly Gln Gly Thr
               100                 105                 110
   Thr Val Thr
           115
<210> 22
   <211> 293
   <212> DNA
   <213> Artificial Sequence
<220>
   <223> Description of Artificial Sequence: Synthetic polynucleotide
<220>
   <221> CDS
   <222> (3)..(293)
<400> 22
   ag gtg cag ctg gtg cag tct ggg gct gag gtg aag aag cct ggg gcc     47
      Val Gln Leu Val Gln Ser Gly Ala Glu Val Lys Lys Pro Gly Ala
      1               5                   10                  15
   tca gtg aag gtc tcc tgc aag gct tct gga tac acc ttc acc ggc tac    95
   Ser Val Lys Val Ser Cys Lys Ala Ser Gly Tyr Thr Phe Thr Gly Tyr
                   20                  25                  30
   tat atg cac tgg gtg cga cag gcc cct gga caa ggg ctt gag tgg atg    143
   Tyr Met His Trp Val Arg Gln Ala Pro Gly Gln Gly Leu Glu Trp Met
               35                  40                  45
   gga tgg atc aac cct aac agt ggt ggc aca aac tat gca cag aag ttt    191
   Gly Trp Ile Asn Pro Asn Ser Gly Gly Thr Asn Tyr Ala Gln Lys Phe
           50                  55                  60
   cag ggc agg gtc acc atg acc agg gac acg tcc atc agc aca gcc tac    239
   Gln Gly Arg Val Thr Met Thr Arg Asp Thr Ser Ile Ser Thr Ala Tyr
       65                  70                  75
   atg gag ctg agc agg ctg aga tct gac gac acg gcc gtg tat tac tgt    287
   Met Glu Leu Ser Arg Leu Arg Ser Asp Asp Thr Ala Val Tyr Tyr Cys
   80                  85                  90                  95
   gcg aga                                                            293
   Ala Arg
<210> 23
   <211> 97
   <212> PRT
   <213> Artificial Sequence
<220>
   <223> Description of Artificial Sequence: Synthetic polypeptide
<400> 23
   Val Gln Leu Val Gln Ser Gly Ala Glu Val Lys Lys Pro Gly Ala Ser
   1               5                   10                  15
   Val Lys Val Ser Cys Lys Ala Ser Gly Tyr Thr Phe Thr Gly Tyr Tyr
               20                  25                  30
   Met His Trp Val Arg Gln Ala Pro Gly Gln Gly Leu Glu Trp Met Gly
           35                  40                  45
   Trp Ile Asn Pro Asn Ser Gly Gly Thr Asn Tyr Ala Gln Lys Phe Gln
       50                  55                  60
   Gly Arg Val Thr Met Thr Arg Asp Thr Ser Ile Ser Thr Ala Tyr Met
   65                  70                  75                  80
   Glu Leu Ser Arg Leu Arg Ser Asp Asp Thr Ala Val Tyr Tyr Cys Ala
                   85                  90                  95
   Arg
<210> 24
   <211> 293
   <212> DNA
   <213> Artificial Sequence
<220>
   <223> Description of Artificial Sequence: Synthetic polynucleotide
<220>
   <221> modified_base
   <222> (112)..(112)
   <223> a, c, t, g, unknown or other
<400> 24
   aggtgcagct ggtgcagtct ggggctgagg tgaagaagct tggggcctca gtgaaggtct    60
   cctgcaaggc ttctggatac accttcaccg gctactatat gcactgggtg cnacaggccc   120
   ctggacaagg gcttgagtgg atgggatgga tcaaccctaa cagtggtggc acaaactatg   180
   cacagaagtt tcagggcagg gtcaccatga ccagggacac gtccatcagc acagcctaca   240
   tggagctgag caggctgaga tctgacgaca cggccgtgta ttactgtgcg aga          293
<210> 25
   <211> 293
   <212> DNA
   <213> Artificial Sequence
<220>
   <223> Description of Artificial Sequence: Synthetic polynucleotide
<400> 25
   aggtgcagct ggtgcagtct ggggctgagg tgaagaagcc tggggcctca gtgaaggtct      60
   cctgcaaggc ttctggatac accttcaccg gctactatat gcactgggtg cgacaggccc     120
   ctggacaagg gcttgagtgg atgggatgga tcaaccctaa cagtggtggc acaaactatg     180
   cacagaagtt tcagggctgg gtcaccatga ccagggacac gtccatcagc acagcctaca     240
   tggagctgag caggctgaga tctgacgaca cggccgtgta ttactgtgcg aga            293
<210> 26
   <211> 10
   <212> DNA
   <213> Artificial Sequence
<220>
   <223> Description of Artificial Sequence: Synthetic oligonucleotide
<400> 26
   tatgattacg 10
<210> 27
   <211> 10
   <212> DNA
   <213> Artificial Sequence
<220>
   <223> Description of Artificial Sequence: Synthetic oligonucleotide
<400> 27
   tatgattacg 10
<210> 28
   <211> 10
   <212> DNA
   <213> Artificial Sequence
<220>
   <223> Description of Artificial Sequence: Synthetic oligonucleotide
<400> 28
   ctaactgggg 10
<210> 29
   <211> 24
   <212> DNA
   <213> Artificial Sequence
<220>
   <223> Description of Artificial Sequence: Synthetic oligonucleotide
<400> 29
   ctggggccaa gggaccacgg tcac 24
<210> 30
   <211> 32
   <212> DNA
   <213> Artificial Sequence
<220>
   <223> Description of Artificial Sequence: Synthetic oligonucleotide
<400> 30
   tttgactact ggggccaagg gaccctggtc ac 32
<210> 31
   <211> 293
   <212> DNA
   <213> Artificial Sequence
<220>
   <223> Description of Artificial Sequence: Synthetic polynucleotide
<400> 31
   aggttcagct ggtgcagtct ggagctgagg tgaagaagcc tggggcctca gtgaaggtct      60
   cctgcaaggc ttctggttac acctttacca gctatggtat cagctgggtg cgacaggccc     120
   ctggacaagg gcttgagtgg atgggatgga tcagcgctta caatggtaac acaaactatg     180
   cacagaagct ccagggcaga gtcaccatga ccacagacac atccacgagc acagcctaca     240
   tggagctgag gagcctgaga tctgacgaca cggccgtgta ttactgtgcg aga            293
<210> 32
   <211> 293
   <212> DNA
   <213> Artificial Sequence
<220>
   <223> Description of Artificial Sequence: Synthetic polynucleotide
<400> 32
   aggtccagct tgtgcagtct ggggctgagg tgaagaagcc tggggcctca gtgaaggttt      60
   cctgcaaggc ttctggatac accttcacta gctatgctat gcattgggtg cgccaggccc     120
   ccggacaaag gcttgagtgg atgggatgga tcaacgctgg caatggtaac acaaaatatt     180
   cacagaagtt ccagggcaga gtcaccatta ccagggacac atccgcgagc acagcctaca     240
   tggagctgag cagcctgaga tctgaagaca cggctgtgta ttactgtgcg aga            293
<210> 33
   <211> 293
   <212> DNA
   <213> Artificial Sequence
<220>
   <223> Description of Artificial Sequence: Synthetic polynucleotide
<400> 33
   aggttcagct ggtgcagtct ggggctgagg tgaagaagcc tggggcctca gtgaaggttt 60
   cctgcaaggc ttctggatac accttcacta gctatgctat gcattgggtg cgccaggccc 120
   ccggacaaag gcttgagtgg atgggatgga gcaacgctgg caatggtaac acaaaatatt 180
   cacaggagtt ccagggcaga gtcaccatta ccagggacac atccgcgagc acagcctaca 240
   tggagctgag cagcctgaga tctgaggaca tggctgtgta ttactgtgcg aga 293
<210> 34
   <211> 292
   <212> DNA
   <213> Artificial Sequence
<220>
   <223> Description of Artificial Sequence: Synthetic polynucleotide
<400> 34
   gaggtccagc tggtacagtc tggggctgag gtgaagaagc ctggggctac agtgaaaatc 60
   tcctgcaagg tttctggata caccttcacc gactactaca tgcactgggt gcaacaggcc 120
   cctggaaaag ggcttgagtg gatgggactt gttgatcctg aagatggtga aacaatatac 180
   gcagagaagt tccagggcag agtcaccata accgcggaca cgtctacaga cacagcctac 240
   atggagctga gcagcctgag atctgaggac acggccgtgt attactgtgc aa 292
<210> 35
   <211> 293
   <212> DNA
   <213> Artificial Sequence
<220>
   <223> Description of Artificial Sequence: Synthetic polynucleotide
<400> 35
   aggtccagct ggtgcagtct ggggctgagg tgaagaagcc tgggtcctca gtgaaggtct 60
   cctgcaaggc ttctggaggc accttcagca gctatgctat cagctgggtg cgacaggccc 120
   ctggacaagg gcttgagtgg atgggaggga tcatccctat ccttggtata gcaaactacg 180
   cacagaagtt ccagggcaga gtcacgatta ccgcggacaa atccacgagc acagcctaca 240
   tggagctgag cagcctgaga tctgaggaca cggccgtgta ttactgtgcg aga 293
<210> 36
   <211> 293
   <212> DNA
   <213> Artificial Sequence
<220>
   <223> Description of Artificial Sequence: Synthetic polynucleotide
<400> 36
   aggtgcagct ggtgcagtct ggggctgagg tgaagaagcc tggggcctca gtgaaggttt 60
   cctgcaaggc atctggatac accttcacca gctactatat gcactgggtg cgacaggccc 120
   ctggacaagg gcttgagtgg atgggaataa tcaaccctag tggtggtagc acaagctacg 180
   cacagaagtt ccagggcaga gtcaccatga ccagggacac gtccacgagc acagtctaca 240
   tggagctgag cagcctgaga tctgaggaca cggccgtgta ttactgtgct aga 293
<210> 37
   <211> 293
   <212> DNA
   <213> Artificial Sequence
<220>
   <223> Description of Artificial Sequence: Synthetic polynucleotide
<400> 37
   aggtgcagct ggtgcagtct ggggctgagg tgaagaagcc tggggcctca gtgaaggttt 60
   cctgcaaggc atctggatac accttcacca gctactatat gcactgggtg cgacaggccc 120
   ctggacaagg gcttgagtgg atgggaataa tcaaccctag tggtggtagc acaagctacg 180
   cacagaagtt ccagggcaga gtcaccatga ccagggacac gtccacgagc acagtctaca 240
   tggagctgag cagcctgaga tctgaggaca cggccgtgta ttactgtgcg aga 293
<210> 38
   <211> 319
   <212> DNA
   <213> Artificial Sequence
<220>
   <223> Description of Artificial Sequence: Synthetic polynucleotide
<220>
   <221> CDS
   <222> (1)..(318)
<400> 38
   gac att ctg atg acc cag tct cca tcc tca ctg tct gca tct ctg gga         48
   Asp Ile Leu Met Thr Gln Ser Pro Ser Ser Leu Ser Ala Ser Leu Gly
   1               5                   10                  15
   ggc aaa gtc acc atc act tgc aag gca agc caa gac att aac aag tat         96
   Gly Lys Val Thr Ile Thr Cys Lys Ala Ser Gln Asp Ile Asn Lys Tyr
               20                  25                  30
   ata gct tgg tac caa cac aag cct gga gaa ggt cct agg ctg ctc ata        144
   Ile Ala Trp Tyr Gln His Lys Pro Gly Glu Gly Pro Arg Leu Leu Ile
           35                  40                  45
   cat tac aca tct aca tta cag cca ggc atc cca tca agg ttc agt gga        192
   His Tyr Thr Ser Thr Leu Gln Pro Gly Ile Pro Ser Arg Phe Ser Gly
       50                  55                  60
   agt ggg tct ggg aga gat tat tcc ttc agc atc acc aac ctg gaa cct        240
   Ser Gly Ser Gly Arg Asp Tyr Ser Phe Ser Ile Thr Asn Leu Glu Pro
   65                  70                  75                  80
   gaa gat att gca act tat tat tgt cta cag tat gat aat ctg tgg acg        288
   Glu Asp Ile Ala Thr Tyr Tyr Cys Leu Gln Tyr Asp Asn Leu Trp Thr
                   85                  90                  95
   ttc ggt gga ggc acc aag ctg gaa atc aaa c                              319
   Phe Gly Gly Gly Thr Lys Leu Glu Ile Lys
               100                 105
<210> 39
   <211> 106
   <212> PRT
   <213> Artificial Sequence
<220>
   <223> Description of Artificial Sequence: Synthetic polypeptide
<400> 39
   Asp Ile Leu Met Thr Gln Ser Pro Ser Ser Leu Ser Ala Ser Leu Gly
   1               5                   10                  15
   Gly Lys Val Thr Ile Thr Cys Lys Ala Ser Gln Asp Ile Asn Lys Tyr
               20                  25                  30
   Ile Ala Trp Tyr Gln His Lys Pro Gly Glu Gly Pro Arg Leu Leu Ile
           35                  40                  45
   His Tyr Thr Ser Thr Leu Gln Pro Gly Ile Pro Ser Arg Phe Ser Gly
       50                  55                  60
   Ser Gly Ser Gly Arg Asp Tyr Ser Phe Ser Ile Thr Asn Leu Glu Pro
   65                  70                  75                  80
   Glu Asp Ile Ala Thr Tyr Tyr Cys Leu Gln Tyr Asp Asn Leu Trp Thr
                   85                  90                  95
   Phe Gly Gly Gly Thr Lys Leu Glu Ile Lys
               100                 105
<210> 40
   <211> 281
   <212> DNA
   <213> Artificial Sequence
<220>
   <223> Description of Artificial Sequence: Synthetic polynucleotide
<220>
   <221> CDS
   <222> (1)..(281)
<400> 40
   gac atc cag atg acc cag tct cca tcc tcc ctg tct gca tct gta gga          48
   Asp Ile Gln Met Thr Gln Ser Pro Ser Ser Leu Ser Ala Ser Val Gly
   1               5                   10                  15
   gac aga gtc acc atc act tgc cag gcg agt cag gac att agc aac tat          96
   Asp Arg Val Thr Ile Thr Cys Gln Ala Ser Gln Asp Ile Ser Asn Tyr
               20                  25                  30
   tta aat tgg tat cag cag aaa cca ggg aaa gcc cct aag ctc ctg atc         144
   Leu Asn Trp Tyr Gln Gln Lys Pro Gly Lys Ala Pro Lys Leu Leu Ile
           35                  40                  45
   tac gat gca tcc aat ttg gaa aca ggg gtc cca tca agg ttc agt gga         192
   Tyr Asp Ala Ser Asn Leu Glu Thr Gly Val Pro Ser Arg Phe Ser Gly
       50                  55                  60
   agt gga tct ggg aca gat ttt act ttc acc atc agc agc ctg cag cct         240
   Ser Gly Ser Gly Thr Asp Phe Thr Phe Thr Ile Ser Ser Leu Gln Pro
   65                  70                  75                  80
   gaa gat att gca aca tat tac tgt caa cag tat gat aat ct                  281
   Glu Asp Ile Ala Thr Tyr Tyr Cys Gln Gln Tyr Asp Asn Leu
                   85                  90
<210> 41
   <211> 94
   <212> PRT
   <213> Artificial Sequence
<220>
   <223> Description of Artificial Sequence: Synthetic polypeptide
<400> 41
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Tyr Asp Asn Leu
                   85                  90
<210> 42
   <211> 281
   <212> DNA
   <213> Artificial Sequence
<220>
   <223> Description of Artificial Sequence: Synthetic polynucleotide
<400> 42
   gacatccaga tgacccagtc tccatcctcc ctgtctgcat ctgtaggaga cagagtcacc      60
   atcacttgcc aggcgagtca ggacattagc aactatttaa attggtatca gcagaaacca     120
   gggaaagccc ctaagctcct gatctacgat gcatccaatt tggaaacagg ggtcccatca     180
   aggttcagtg gaagtggatc tgggacagat tttactttca ccatcagcag cctgcagcct     240
   gaagatattg caacatatta ctgtcaacag tatgataatc t                         281
<210> 43
   <211> 273
   <212> DNA
   <213> Artificial Sequence
<220>
   <223> Description of Artificial Sequence: Synthetic polynucleotide
<400> 43
   gacatccaga tgacccagtc tccatcctcc ctgtctgcat ctgtaggaga cagagtcacc 60
   atcacttgcc gggcgagtca gggcattagc aattatttag cctggtatca gcagaaacca 120
   gggaaagttc ctaagctcct gatctatgct gcatccactt tgcaatcagg ggtcccatct 180
   cggttcagtg gcagtggatc tgggacagat ttcactctca ccatcagcag cctgcagcct 240
   gaagatgttg caacttatta ctgtcaaaag tat 273
<210> 44
   <211> 38
   <212> DNA
   <213> Artificial Sequence
<220>
   <223> Description of Artificial Sequence: Synthetic oligonucleotide
<400> 44
   gtggacgttc ggccaaggga ccaaggtgga aatcaaac
<210> 45
   <211> 34
   <212> DNA
   <213> Artificial Sequence
<220>
   <223> Description of Artificial Sequence: Synthetic oligonucleotide
<400> 45
   acgttcggcg gagggaccaa ggtggagatc aaac
<210> 46
   <211> 275
   <212> DNA
   <213> Artificial Sequence
<220>
   <223> Description of Artificial Sequence: Synthetic polynucleotide
<400> 46
   catccggatg acccagtctc cattctccct gtctgcatct gtaggagaca gagtcaccat 60
   cacttgctgg gccagtcagg gcattagcag ttatttagcc tggtatcagc aaaaaccagc 120
   aaaagcccct aagctcttca tctattatgc atccagtttg caaagtgggg tcccatcaag 180
   gttcagcggc agtggatctg ggacggatta cactctcacc atcagcagcc tgcagcctga 240
   agattttgca acttattact gtcaacagta ttata 275
<210> 47
   <211> 277
   <212> DNA
   <213> Artificial Sequence
<220>
   <223> Description of Artificial Sequence: Synthetic polynucleotide
<400> 47
   gacatccaga tgacccagtc tccatcctcc ctgtctgcat ctgtaggaga cagagtcacc      60
   atcacttgcc gggcgagtca gggcattagc aattctttag cctggtatca gcagaaacca     120
   gggaaagccc ctaagctcct gctctatgct gcatccagat tggaaagtgg ggtcccatcc     180
   aggttcagtg gcagtggatc tgggacggat tacactctca ccatcagcag cctgcagcct     240
   gaagattttg caacttatta ctgtcaacag tattata                              277
<210> 48
   <211> 274
   <212> DNA
   <213> Artificial Sequence
<220>
   <223> Description of Artificial Sequence: Synthetic polynucleotide
<400> 48
   gacatccaga tgacccagtc tccatcctcc ctgtctgcat ctgtaggaga cagagtcacc      60
   atcacttgcc gggcgagtca gggcattagc aattatttag cctggtatca gcagaaacca     120
   gggaaagttc ctaagctcct gatctatgct gcatccgctt tgcaatcagg gggtcccatc     180
   tcggttcagt ggcagtggat ctgggacaga tttcactctc accatcagca gcctgcagcc     240
   tgaagatgtt gcaacttatt actgtcaaaa gtat                                 274
<210> 49
   <211> 277
   <212> DNA
   <213> Artificial Sequence
<220>
   <223> Description of Artificial Sequence: Synthetic polynucleotide
<400> 49
   catccagatg acccagtctc catcctccct gtctgcatct gtaggagaca gagtcaccat      60
   cacttgccgg gcaagtcagg gcattagaaa tgatttaggc tggtatcagc agaaaccagg     120
   gaaagcccct aagctcctga tctatgctgc atccagttta caaagtgggg tcccatcaag     180
   gttcagcggc agtggatctg gcacagattt cactctcacc atcagcagcc tgcagcctga     240
   agattttgca acttattact gtctacaaga ttacaat                              277
<210> 50
   <211> 277
   <212> DNA
   <213> Artificial Sequence
<220>
   <223> Description of Artificial Sequence: Synthetic polynucleotide
<400> 50
   gacatccaga tgacccagtc tccatcctcc ctgtctgcat ctgtaggaga cagagtcacc     60
   atcacttgcc gggcaagtca gggcattaga aatgatttag gctggtatca gcagaaacca    120
   gggaaagccc ctaagcgcct gatctatgct gcatccagtt tgcaaagtgg ggtcccatca    180
   aggttcagcg gcagtggatc tgggacagaa ttcactctca caatcagcaa cctgcagcct    240
   gaagattttg caacttatta ctgtctacag cataata                             277
<210> 51
   <211> 277
   <212> DNA
   <213> Artificial Sequence
<220>
   <223> Description of Artificial Sequence: Synthetic polynucleotide
<400> 51
   gacatccaga tgacccagtc tccatcctca ctgtctgcat ctgtaggaga cagagtcacc     60
   atcacttgtc gggcgagtca gggcattagc aattatttag cctggtttca gcagaaacca    120
   gggaaagccc ctaagtccct gatctatgct gcatccagtt tgcaaagtgg ggtcccatca    180
   aggttcagcg gcagtggatc tgggacagat ttcactctca ccatcagcag cctgcagcct    240
   gaagattttg caacttatta ctgccaacag tataata                             277
<210> 52
   <211> 277
   <212> DNA
   <213> Artificial Sequence
<220>
   <223> Description of Artificial Sequence: Synthetic polynucleotide
<400> 52
   catccagttg acccagtctc catcctccct gtctgcatct gtaggagaca gagtcaccat     60
   cacttgccgg gcaagtcagg gcattagcag tgctttagcc tggtatcagc agaaaccagg    120
   gaaagctcct aagctcctga tctatgatgc ctccagtttg gaaagtgggg tcccatcaag    180
   gttcagcggc agtggatctg ggacagattt cactctcacc atcagcagcc tgcagcctga    240
   agattttgca acttattact gtcaacagtt taataat                             277

## Description

### FIELD OF THE DISCLOSURE

The present disclosure is directed, in part, to methods of detecting the metastatis status of cell types, including without limitation, breast cells.

### BACKGROUND OF THE DISCLOSURE

Breast cancer is the most common cancer among women in the United States, the second most common cause of cancer death, and the main cause of death in women ages 45 to 55 years. In 2009, approximately 192,370 American women were diagnosed with breast cancer, and an estimated 40,170 women died of the disease (Jemal et al. Cancer statistics, 2009. CA Cancer J Clin. 2009:59(4):225-249). Triple negative breast cancer (TNBC) accounts for approximately 15% of breast cancers (Kaplan et al. Impact of triple negative phenotype on breast cancer prognosis. Poster presented at: 29th Annual San Antonio Breast Cancer Symposium; December 14-17, 2006; San Antonio, TX). The term TNBC has recently been coined to describe a subtype of breast cancer that lacks expression of the estrogen receptor (ER) and progesterone receptor (PR) and does not over express human epidermal growth factor 2 receptor (HER2) protein. TNBC is an important area of research for both researchers and clinicians alike because (1) TNBC is a poor prognostic factor for disease-free and overall survival, (2) no effective specific targeted therapy is readily available for TNBC, (3) there is a clustering of TNBC cases in premenopausal women and in women of African descent, and (4) the overlap of BRCA1-associated breast cancers with the TNBC phenotype is significant.

An estimated 1 million cases of breast cancer are diagnosed annually worldwide. Of these, approximately 170,000 are of the triple negative (ER-/PR-/HER2-) phenotype (Anders et al. Biology, metastatic patterns, and treatment of patients with triple negative breast cancer. Clin Breast Cancer. 2009;9(suppl 2):S73-S81). Of these TNBC cases, about 75% are "basal-like" (Rakha et al. Basal-like breast cancer: a critical review. J Clin Oncol. 2008;26(15):2568-2581). The prevalence of TNBC is highest in premenopausal African American women; a recent report notes that 39% of all African American premenopausal women diagnosed with breast cancer are diagnosed with TNBC (Carey et al. Race, breast cancer subtypes, and survival in the Caroline Breast Cancer Study. JAMA. 2006;295(21):2492-2502).

Although the terms basal-like breast cancer and TNBC are often used interchangeably, they are not synonymous. TNBC refers to the immunophenotype of the breast cancer that is immunologically negative to ER, PR, and HER2.

Basal-like breast cancer refers to the molecular phenotype of the tumor that has been defined by cDNA microarrays. Of these TNBCs, about 75% of them are of the basal-like type. Perou et al. (Perou et al. Molecular portraits of human breast tumours. Nature. 2000;406(6797):747-752) described various molecular subtypes or molecular profiles of breast cancers. They described four subtypes based on cDNA microarrays, including a basal-like subtype of breast cancer, and noted that most TNBCs clustered in the basal-like subtype.

The luminal subtypes of breast cancers express high amounts of luminal cytokeratins and express genetic markers of luminal epithelial cells and normal breast cells (Rakha et al. Prognostic markers in triple negative breast cancer. Cancer. 2007;109(1):25-32; Sotiriou et al. Gene-expression signatures in breast cancer. N Engl J Med. 2009:360(8):790-800). In contrast, basal-like breast cancers tend to express cytokeratins associated with basal types of cancers, as they arise from the outer basal layer.

Basal-like breast cancers are typically high-grade and poorly differentiated when examined morphologically. While the TNBC phenotype is defined by immunohistochemistry, no established diagnostic criteria have been identified for basal-like breast cancer on a morphological basis. In general, basal-like breast carcinomas are morphologically consistent with a high nuclear grade, high mitotic count, and necrosis, such as a grade 3 invasive ductal carcinoma, not otherwise specified. Some have the histomorphology of medullary carcinoma or metaplastic carcinoma. It has also been reported that almost 82% of basal-like breast cancers express p53 compared with 13% in the luminal A subgroup (Sorlie et al. Gene expression patterns of breast carcinomas distinguish tumor subclasses with clinical implications. Proc Natl Acad Sci USA. 2001 ;98(19): 10869-10874).

A subset of TNBC and basal-like breast cancer that is of low histological grade includes secretory, adenoid cystic, acinic cell, and apocrine breast carcinoma. Useful immunohistochemical markers for characterizing basal-like carcinomas are CK₅, CK6, CK14, CK8/CK18, p63, P-cadherin, vimentin, epidermal growth factor receptor 1 (EGFR1 [or HERi]), c-kit, and other growth factors such as insulin-like growth factor receptor (IGFR) (Rakha et al. Basal-like breast cancer: a critical review. J Clin Oncol. 2008:26(15):2568-2581; Sotiriou et al. Gene-expression signatures in breast cancer. N Engl J Med.2009;360(8):790-800; Korsching et al. Basal carcinoma of the breast revisited: an old entity with new interpretations. J Clin Pathol. 2008;61(5):553-560).

D2 (Korkola et al 2007 - BMC Cancer 2007 vol 7 page 61) discloses identification of a robust gene signature that predicts breast cancer outcome in independent data sets.

D3 (Hannemann et al 2005 - J Clin Oncol. 2005 May 20;23(15):3331-42.) discloses changes in gene expression associated with response to neoadjuvant chemotherapy in breast cancer.

There is a need to develop prognostic and diagnostic tests that are capable of determining the metastatic status of cancer cells, particularly triple negative breast cancer cells.

### SUMMARY OF THE DISCLOSURE

In one aspect is provided a method of predicting disease progression comprising: determining the expression of glia maturation factor beta in an obtained sample of breast tissue, wherein expression of glia maturation factor beta is indicative of lymph node metastatis.

In another aspect is provided a method of predicting disease progression comprising: determining the expression of glia maturation factor beta in an obtained sample of breast tissue, wherein expression of glia maturation factor beta is indicative of an untreated or treated prognosis that is reduced compared to an absence of said expression.

In another aspect is provided a method of predicting disease progression comprising: determining the expression of glia maturation factor beta in an obtained sample of breast tissue, wherein non-expression of glia maturation factor beta is indicative of an enhanced untreated or treated prognosis compared to the presence of said expression.

In another aspect is provided a method as described above, wherein said tissue is obtained from a stage IIB breast cancer.

In another aspect is provided a method as described above, wherein said determining is obtained with an antibody to glia maturation factor beta.

In another aspect is provided a method as described above, wherein said antibody shows nuclear staining.

In another aspect is provided a method as described above, wherein said antibody shows cytoplasmic staining. Suitably said antibody further shows nuclear staining.

In another aspect is provided a method as described above, wherein said expression is associated with selecting a clinical approach.

In another aspect is provided a method as described above, wherein said breast cancer is obtained from a tumor larger than 5 cm.

In another aspect is provided a method as described above, wherein said lymph node metastatis is confirmed by biopsy.

In another aspect is provided a method as described above, wherein the breast tissue is estrogen receptor negative, progesterone receptor negative and does not over-express human epidermal growth factor 2 receptor protein.

In another aspect is provided an method for detecting a GMB-B antigen which binds to an antibody or antibody fragment specific for a GMF-B antigen, comprising one or more of the heavy- chain CDR antigen binding site sequences selected from the group consisting of SEQ ID NOs: 2, 3, and 4, and one or more of the light-chain CDR antigen binding site sequences selected from the group consisting of SEQ ID NOs: 6, 7, and 8, comprising:
(a) contacting a sample of breast tissue, wherein the breast tissue is estrogen receptor negative, progesterone receptor negative and does not over-express human epidermal growth factor 2 receptor protein, with an effective binding amount of said antibody or antibody fragment; and
(b) detecting said antigen by detecting the binding of the antibody to the GMF-B antigen, wherein said binding is prognostic of disease progression.

### BRIEF DESCRIPTION OF THE DRAWINGS

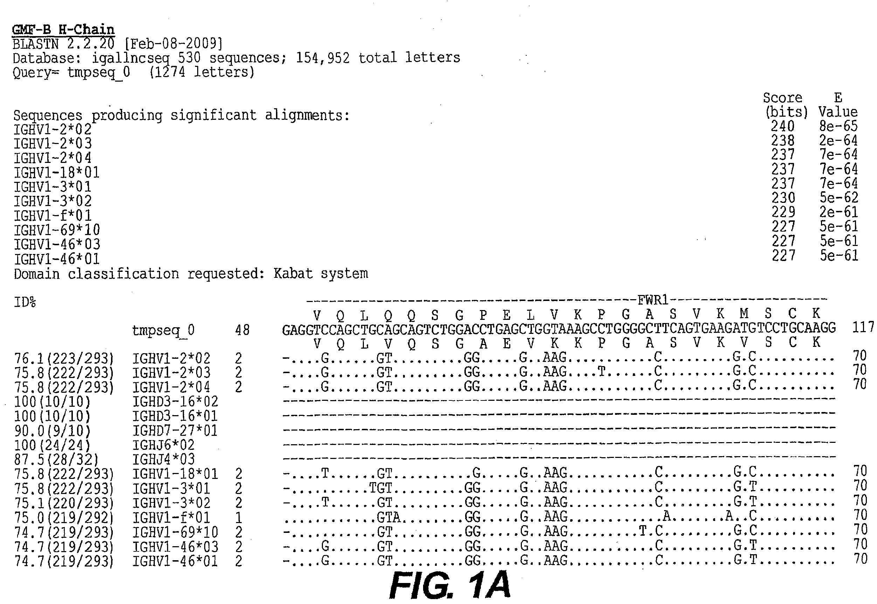

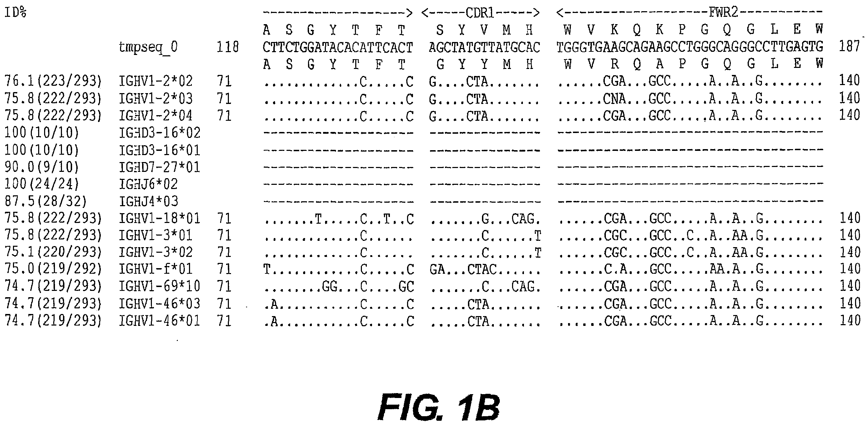

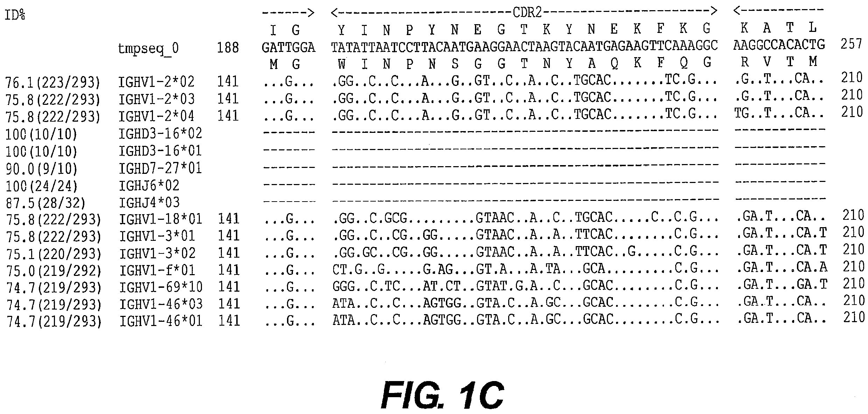

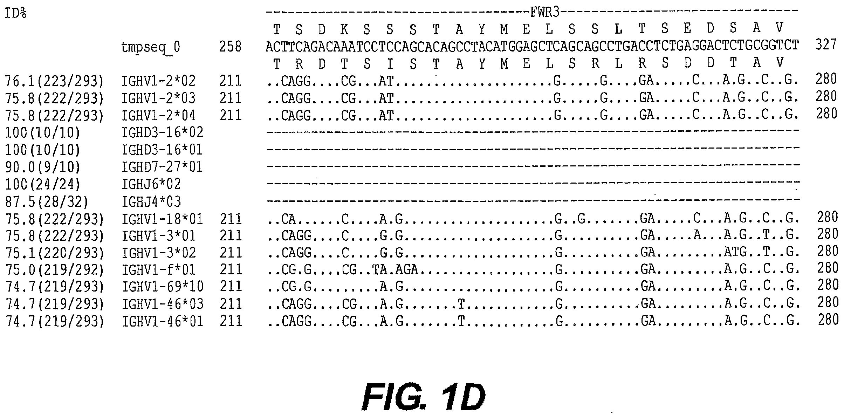

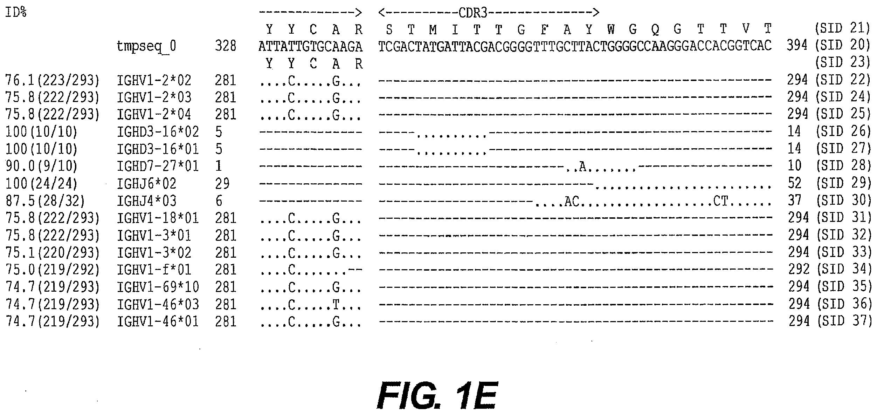

Figures 1A-1E. Alper-sGMF-B mAb heavy chain sequence information. FWRs and CDRs of the heavy chain of a Alper-sGMF-B mAb, in which the polypeptide sequence provided in the top line (SEQ ID NO: 21) corresponds to the sequence of the Alper-sGMF-B mAb. Amino acid residues are numbered using the convention of Kabat et al., (1991) Sequences of Proteins of Immunological Interest, 5th Edition, Department of Health and Human Services, Public Health Service, National Institutes of Health, Bethesda (NIH Publication No. 91-3242). Bold residues set forth in underlined text indicate specificity determining residues (SDRs). Figure 1 also discloses SEQ ID NOS 20, 23, 22, and 24-37, respectively, in order of appearance.

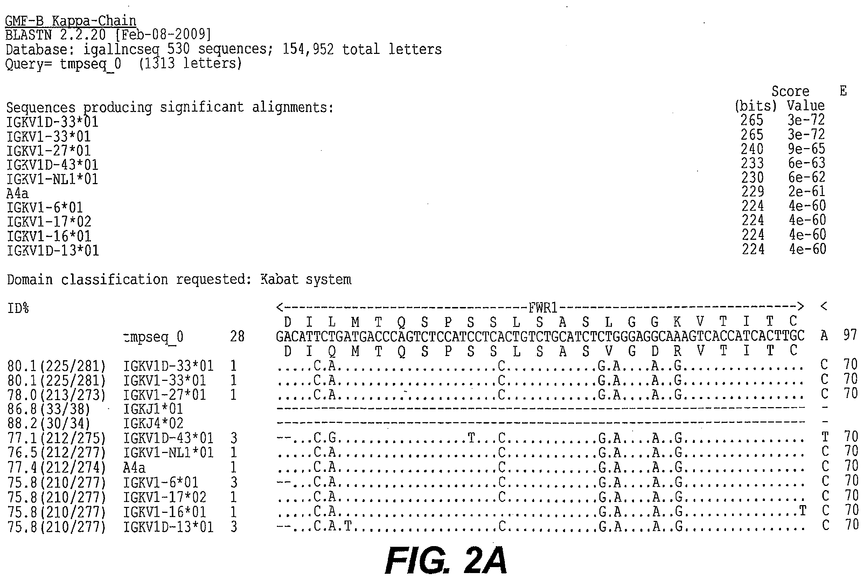

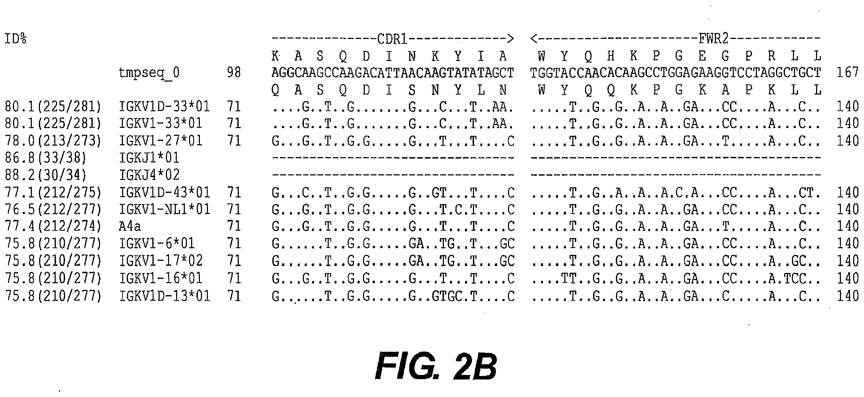

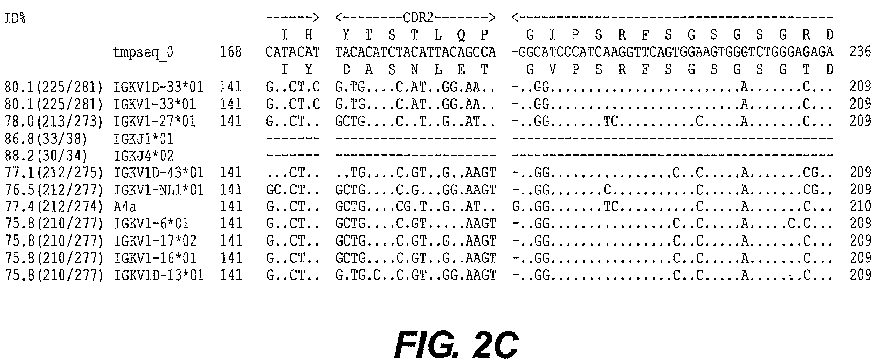

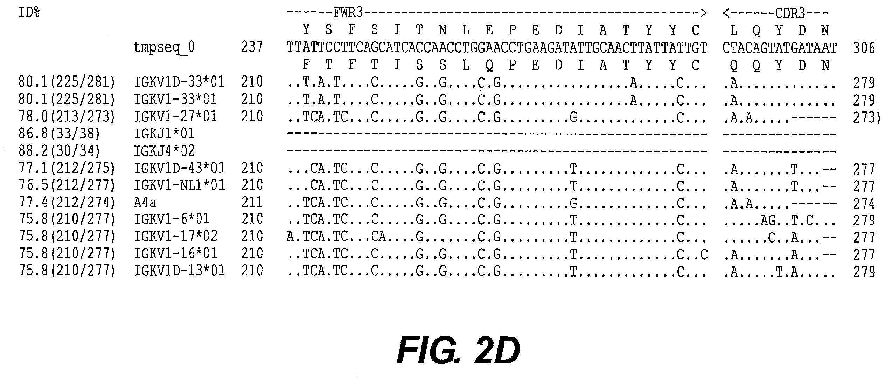

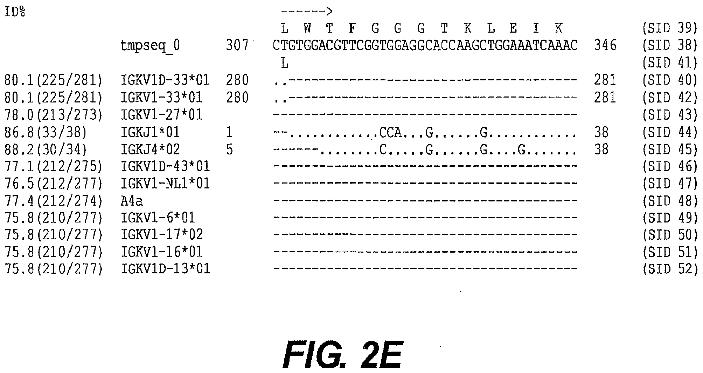

Figures 2A-2E. Alper-sGMF-B mAb light chain sequence information. FWRs and CDRs of the light chain of a Alper-sGMF-B mAb, in which the polypeptide sequence provided in the top line (SEQ ID NO: 39) corresponds to the sequence of the Alper-sGMF-B mAb. Amino acid residues are numbered using the convention of Kabat *et al.* Bold residues set forth in underlined text indicate the specificity determining residues (SDRs). Figure 2 also discloses SEQ ID NOS 38, 41, 40, and 42-52, respectively, in order of appearance.

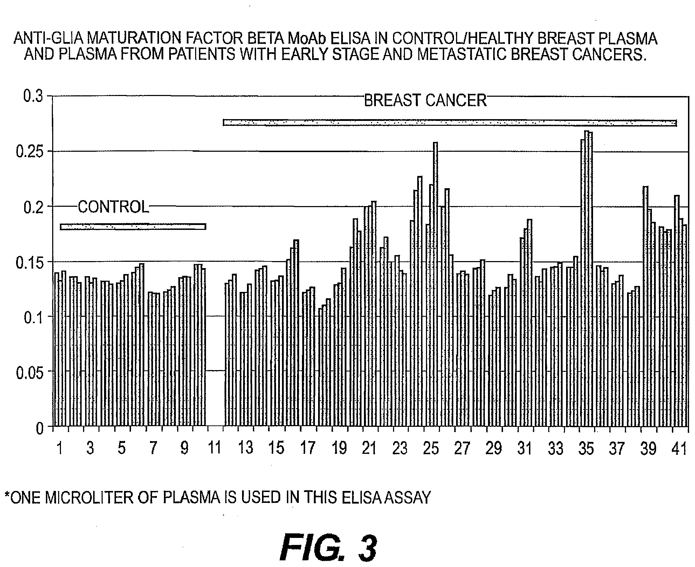

Figure 3. Breast Cancer Patient GMF-B Levels

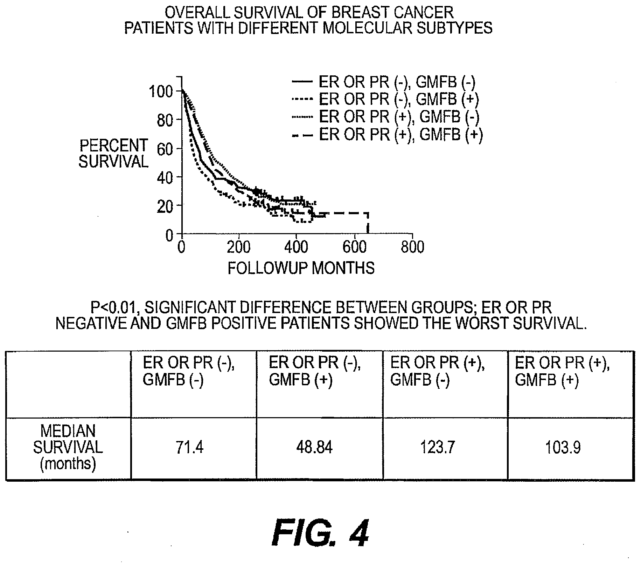

Figure 4: Overall survival of breast cancer patients with different molecular subtypes.

### BRIEF DESCRIPTION OF CERTAIN SEQUENCES

SEQ ID NOs: 1 and 21 show the amino acid sequence of Alper-sGMF-B mAb Heavy Chain (see Figure 1)
SEQ ID NO: 2 shows CDR1 of Alper-sGMF-B mAb Heavy Chain
SEQ ID NO: 3 shows CDR2 of Alper-sGMF-B mAb Heavy Chain
SEQ ID NO: 4 shows CDR3 of Alper-sGMF-B mAb Heavy Chain
SEQ ID NOs: 5 and 39 show the amino acid sequence of Alper-sGMF-B mAb Kappa Chain (see Figure 2)
SEQ ID NO: 6 shows CDR1 of Alper-sGMF-B mAb Kappa Chain
SEQ ID NO: 7 shows CDR2 of Alper-sGMF-B mAb Kappa Chain
SEQ ID NO: 8 shows CDR3 of Alper-sGMF-B mAb Kappa Chain
SEQ ID NO: 9 shows Full-length GMF-B Antigen
SEQ ID NO: 10 shows Full-length GMF-B Antigen without Methionine
SEQ ID NO: 11 shows Processed GMF-B Antigen
SEQ ID NO: 12 shows Processed GMF-B Antigen without Methionine
SEQ ID NOs: 13 and 20 show the nucleotide sequence of Alper-sGMF-B mAb Heavy Chain (see Figure 1)
SEQ ID NOs: 14 and 38 shows the nucleotide sequence of Alper-sGMF-B mAb Kappa Chain (see Figure 2)
SEQ ID NO: 15 shows the amino acid sequence of Epitope 1 of GMF-B
SEQ ID NO: 16 shows the amino acid sequence of Epitope 2 of GMF-B
SEQ ID NO: 17 shows the amino acid sequence of Epitope 3 of GMF-B

### DETAILED DESCRIPTION

### 1. Definitions

Antibody: This refers to single chain, two-chain, and multi-chain proteins and glycoproteins belonging to the classes of polyclonal, monoclonal, chimeric and hetero immunoglobulins (monoclonal antibodies being preferred); it also includes synthetic and genetically engineered variants of these immunoglobulins. "Antibody fragment" includes Fab, Fab', F(ab')₂, and Fv fragments, as well as any portion of an antibody having specificity toward a desired target epitope or epitopes.

Monoclonal Antibody: This refers to antibodies that are identical because they are produced by one type of immune cell that are all clones of a single parent cell. The monoclonal antibodies of the present invention can include intact monoclonal antibodies, antibody fragments, conjugates, or fusion proteins, which contain a V_{H} and a V_{L} where the CDRs form the antigen binding site.

Chimeric Antibody: This refers to an antibody which includes sequences derived from two different antibodies, which typically are of different species. Most typically, chimeric antibodies include human and non-human antibody fragments, generally human constant and non-human variable regions. Humanized antibodies can or cannot be considered chimeric.

Humanized Antibody: This refers to an antibody derived from a non-human antibody. The humanized antibody retains or substantially retains the antigen-binding properties of the parent antibody but is less immunogenic in humans than its parent antibody.

Antibody Conjugates, Fusion Proteins, and Bispecific Antibodies: These refer to monoclonal antibodies conjugated by chemical methods with radionuclides, drugs, macromolecules, or other agents.

Antigen: This refers to one or more molecules or one or more portions of a molecule capable of being bound by an antibody which is additionally capable of inducing an animal to produce an antibody capable of binding to an epitope of that antigen. An antigen can have one or more than one epitope. The specific reaction referred to above is meant to indicate that the antigen will react, in a highly preferential manner, with its corresponding antibody and not with the multitude of other antibodies which can be evoked by other antigens. The binding of antigen to antibody must be above background levels.

Epitope: This refers to that portion of any molecule capable of being recognized by, and bound by, an antibody. In general, epitopes consist of chemically active surface groupings of molecules, for example, amino acids or sugar side chains, and have specific three-dimensional structural characteristics as well as specific charge characteristics. The epitopes of interest for the present invention are epitopes comprising amino acids.

Complementarity Determining Region, or CDR: This refers to amino acid sequences which together define the binding affinity and specificity of the natural Fv region of a native immunoglobulin binding site. The light and heavy chains of an immunoglobulin each have three CDRs. By definition, the CDRs of the light chain are bounded by the residues at positions 24 and 34 (CDR1), 50 and 56 (CDR2), 88 and 94 (CDR3); the CDRs of the heavy chain are bounded by the residues at positions 36 and 44 (CDR1), 49-65 (CDR2), and 108-117 (CDR3), using the numbering convention delineated by Kabat et al., (1991) Sequences of Proteins of Immunological Interest, 5th Edition, Department of Health and Human Services, Public Health Service, National Institutes of Health, Bethesda (NIH Publication No. 91-3242).

Framework Region or FWR: This refers to amino acid sequences interposed between CDRs. These portions of the antibody serve to hold the CDRs in an appropriate orientation for antigen binding.

Specificity Determining Residue, or SDR: This refers to amino acid residues that can be unique to Alper-sGMF-B mAb when compared to other IgGs. Preferentially, the SDR is the part of an immunoglobulin that is directly involved in antigen contact.

Constant Region: This refers to the portion of an antibody molecule which confers effector functions. A heavy chain constant region can be selected from any of five isotypes: alpha, delta, epsilon, gamma or mu. Heavy chains of various subclasses (such as the IgG subclass of heavy chains) are responsible for different effector functions. Thus, by choosing the desired heavy chain constant region, humanized antibodies with the desired effector function can be produced. A light chain constant region can be of the kappa or lambda type, preferably the kappa type.

Immunogenicity: A measure of the ability of a targeting protein or therapeutic moiety to elicit an immune response (humoral or cellular) when administered to a recipient. The present invention is concerned with the immunogenicity of antibodies to GMF-B.

Immunoreactivity: A measure of the ability of an immunoglobulin to recognize and bind to a specific antigen.

GMF-B Antibodies or GMF-B mAbs: This refers to antibodies preferential to expression products of the GMF-B gene and homologues of the GMF-B gene, which can include antibodies specific to modified forms of the expression product that are produced by cancer cells. The antibodies include variants, such as chimeric, humanized, and other variants known to those skilled in the art. GMF-B antibodies are said to be specific for the GMF-B antigen if they exhibit preferential binding to a GMF-B antigen at least 85% of the time, at least 90% of the time, or, in a preferred aspect, at least 95% of the time relative to any other protein.

GMF-B Antigens: This refers to expression products generated by *GMF-B,* which can be used as antigens, target molecules, biomarkers, or any combination thereof. A GMF-B antigen can be produced by the *GMF-B* gene and homologues of the *GMF-B* gene, and can include various modifications introduced by the cells expressing a GMF-B antigen, such as cancer cells.

Substantially Similar Binding Properties: This refers to a chimeric antibody, such as a humanized antibody or fragments thereof which retain the ability to preferentially bind an antigen recognized by the parent antibody used to produce the chimeric antibody, such as a humanized antibody, or fragments thereof. Preferably, the affinity of a chimeric antibody, humanized antibody, or antibody fragment is at least about 10% of the affinity of the parent antibody, more preferably at least about 25%, even more preferably at least about 50%. Most preferably, a chimeric antibody, preferably a humanized antibody, or antibody fragments thereof exhibit an antigen-binding affinity that is at least about 75% of the affinity of the parent antibody. Methods for assaying antigen-binding affinity are known in the art and include half-maximal binding assays, competition assays, and Scatchard analysis. In a preferred aspect, antigen-binding affinity is assayed using a competition assay.

Substantially Homologous: Refers to immunoglobulin sequences that exhibit at least about 85% identity, more preferably about 90% identity, most preferably about 95% identity with a reference immunoglobulin sequence, where % identity is determined by comparing the number identical of amino acid residues between the two immunoglobulins, where the positions of the amino acid residues are indicated using the Kabat numbering scheme.

Sameness for Monoclonal Antibody Products: For the purpose of determining sameness of monoclonal antibodies, and products thereof, the complementarity determining regions of the heavy and light chain variable regions are the principal molecular structural feature of a monoclonal antibody product. Two monoclonal antibodies can be considered the same if the amino acid sequences of the CDRs were the same, or if there were only minor amino acid differences between them. Whether differences in the amino acid sequences are minor can be determined by factors that include (but are not limited to) whether any particular residues have been established to be important for antigen binding, such as to be a Specificity Determining Residue. Amino acid differences outside the CDRs, or differences due to glycosylation patterns or post translational modifications do not result in different monoclonal antibodies. Changes in antibody structure that do not constitute differences between two monoclonal antibody products with the same CDRs include changes in the FWRs (i.e., humanizing a non-human derived monoclonal antibody or engineering certain framework residues that are important for antigen contact or for stabilizing the binding site, or changes in the constant region (i.e., changing the class or subclass of the constant region, changing specific amino acid residues which might alter an effector function, or changing the species from which the constant region is derived).

Substantially pure: For the purpose of the present invention, substantially pure refers to a homogeneous preparation preferably of a GMF-B antibody or antibody fragment, or other chemical or biological agents. Substantially pure immunoglobulins of at least 80% homogeneity are preferred, with about 90% to about 95% homogeneity being more preferred, and 98% to 99% or more homogeneity is most preferred, and is generally considered acceptable for pharmaceutical uses.

Triple negative breast cancer: As used herein, triple negative breast cancer (TNBC) is a subtype of breast cancer that overlaps with basal-like breast cancer. TNBC is defined by a lack of detectable protein expression of the estrogen receptor (ER) and progesterone receptor (PR) and the absence of HER2 protein over expression. TNBC refers to the immunophenotype of that is immunologically negative to ER, PR and HER2.

### 2. Prognostic Methods, Diagnostic Methods, Assays, and Kits

Also disclosed is an immunoassay for preferentially detecting a sGMF-B antigen comprising an antibody or antibody fragment of the present invention.

Also disclosed is an immunoassay for preferentially detecting one or more GMF-B antigens, including a sGMF-B antigen, which bind to a monoclonal antibody having one or more of the heavy chain CDR antigen binding site amino acid sequences set forth in Figure 1, and one or more of the light chain CDR antigen binding site amino acid sequences set forth in Figure 2.

Such immunoassays can be used in any suitable manner, including, without limitation, by comprising: (a) contacting the sample with an effective binding amount of one of the antibodies or antibody fragments of the invention; and (b) detecting the antigen by detecting
the binding of the antibody to a GMF-B antigen. Immunoassays can be used to detect cancer cells expressing a GMF-B antigen.

In yet another aspect, the present disclosure provides an immunoassay for monitoring a GMB-B antigen which binds to an antibody or antibody fragment specific for a GMF-B antigen, comprising one or more of the heavy-chain CDR antigen binding site sequences selected from the group consisting of SEQ ID NOs: 2, 3, and 4, and one or more of the light-chain CDR antigen binding site sequences selected from the group consisting of SEQ ID NOs: 6, 7, and 8, comprising: (a) contacting a sample with an effective binding amount of the antibody or antibody fragment; and (b) detecting the antigen by detecting the binding of the antibody to the GMF-B antigen, wherein the binding is prognostic of disease progression.

In one aspect, the breast cancer or tissue might be any breast cancer or tissue. In another aspect, the breast cancer is triple negative breast cancer. In an aspect, the breast cancer is at stage IIB. In a further aspect, the breast cancer is at stage IIB and is triple negative.

In one aspect, the breast cancer is a basal tumor. In another aspect, it is a luminal tumor. As used herein, a stage IIB breast cancer is when the cancer is between 2 and 5 centimeters in size and has spread to the lymph nodes under the arm or the cancer is larger than 5 centimeters but has not spread to the lymph nodes under the arm. In another aspect, ovarian cancer can be evaluated.

Antibodies and antibody fragments can be used in immunoassays to screen body fluids, such as serum, sputum, effusions, urine, cerebrospinal fluid, and the like, for the presence of sGMF-B. Antibodies and antibody fragments can be used for scanning or radioimaging, when labeled with an appropriate radiolabel, to detect primary or metastatic foci of tumor cells. Furthermore, the antibodies are useful in lymphoscintigraphy to detect lymph node involvement in the disease.

Antibodies or antibody fragments are useful for immunoassays of the present invention which detect or quantitate GMF-B or cells bearing GMF-B in a sample. Such an immunoassay typically comprises incubating a biological sample from a subject with a need therefor in the presence of a detectably labeled antibody capable of identifying the tumor antigen, and detecting the labeled antibody which is bound in a sample.

In one aspect, a status of a cell or collection of cells can be determined using an antibody as disclosed or of fragment thereof whether that cell, collection of cells, sample etc. are metastatic tumor cells, non-metastatic tumor cells, from a solid tumor or normal cells. A status of a subject can include whether the analysis provides information on whether a metastatic cancer or non-metastatic tumor is present in the subject.

Examples of confirmatory analysis, assays, tests, such as histological examination of samples, and so forth that can be used to confirm or in combination with those disclosed herein include, without limitation, those set forth in Alper, US Publication No. 2008/0293162.

In an aspect of the present invention the level, localization or both of one or more forms of GMF-B, including sGMF-B, is diagnostic or prognostic of a disease or outcome probability.

In an aspect of the present invention a reduced level of sGMF-B in a cell, collection of cells or sample can diagnose, prognose, determine, confirm or indicate that such derived is from a metastatic tissue. In one aspect, "reduced" can mean reduced relative to a control, with the control being a normal cell of the same type that is non-metastatic. In this aspect, the reduction can be greater than 10, 20, 30, 40, 50, 60, 70, 80, 90, 95, or 99%.

In an aspect of the present invention, a similar level of sGMF-B in a cell, collection of cells or sample to a normal control can diagnose, prognose, determine, confirm or indicate that such cell was derived from a non-metastatic tissue.

In an aspect of the present invention, a lack of localization of sGMF-B in a cell nucleus can diagnose, prognose, determine, confirm or indicate that such derived is from a metastatic tissue.

In an aspect of the present invention, localization of sGMF-B in a cell, collection of cells or sample to a normal control can diagnose, prognose, determine, confirm or indicate that such derived from a non-metastatic tissue.

In an aspect of the present invention, the cell, collection of cells or sample is a cervical or breast cell collection of cells or sample, in particular human breast cells.

Antibodies and antibody fragments are also useful for immunopathological analysis, such as the differential diagnosis of tumor type, and the subclassification of the tumor based on its expression or localization of at least one form of GMF-B, including sGMF-B, including, without limitation, assessment of metastatic potential, predicted responses to therapy, and overall prognosis.

GMF-B antibodies and antibody fragments permit the definition of subpopulations of tumor cells among the heterogeneous cells present in a growing tumor and can be used, for example, in the typing and cross-matching of the tumor cell "lines," including, without limitation, by means of flow cytometry, both at the time of surgery and prior to therapy. An analysis of the tumor cell populations or subpopulations with antibodies or antibody fragments of this invention, and a battery of additional antibodies or antibody fragments, can be used to define (a) which antigen preparation would be the most appropriate for specific active immunotherapy, (b) which antibody or antibody fragment or chimeric antibody would be efficacious for the particular cancer; and (c) which antibody or combination of antibodies or antibody fragments should be used for imaging the patient at a later date in search for recurrent or metastatic tumors.

A biological sample can be treated with nitrocellulose, or other solid support or carrier which is capable of immobilizing cells, cell particles or soluble proteins or glycoproteins. The support can then be washed with suitable buffers followed by treatment with the detectably labeled antibody. The solid phase support can then be washed with the buffer a second time to remove unbound antibody. The amount of bound label on the solid support can then be detected by conventional means.

One of the ways in which the antibody can be detectably labeled is by linking the same to an enzyme and use in an enzyme immunoassay (EIA) or enzyme-linked immunosorbent assay (ELISA). This enzyme, when subsequently exposed to its substrate, will react with the substrate generating a chemical moiety which can be detected, for example, by spectrophotometric, fluorometric or by visual means. In an alternate embodiment, the enzyme is used to label a binding partner for the antibody. Such a binding partner can be an antibody against the constant or variable region of the antibody, such as a heterologous anti-mouse immunoglobulin antibody. Alternatively, the binding partner can be a non-antibody protein capable of binding to the antibody.

By radioactively labeling the antibodies, it is possible to detect GMF-B through the use of a radioimmunoassay (RIA). The radioactive isotope can be detected by such means as the use of a gamma counter or a scintillation counter or by autoradiography. Isotopes which are particularly useful for the purpose of the present invention are known in the art.

It is also possible to label the antibodies with a fluorescent compound. When the fluorescently labeled antibody is exposed to light of the proper wave length, its presence can then be detected due to fluorescence. The antibodies also can be detectably labeled by coupling to a chemiluminescent compound. The presence of the chemiluminescently labeled antibody is then determined by detecting the presence of luminescence that arises during the course of a chemical reaction. A bioluminescent compound can also be used to label the antibodies. Bioluminescence is a type of chemiluminescence found in biological systems, in which a catalytic protein increases the efficiency of the chemiluminescent reaction. The presence of a bioluminescent protein is determined by detecting the presence of luminescence. Important bioluminescent compounds for purposes of labeling are luciferin, luciferase and sequorin.

Detection of the antibody, fragment or derivative can be accomplished by a scintillation counter, for example, if the detectable label is a radioactive gamma emitter, or by a fluorometer, for example, if the label is a fluorescent material. In the case of an enzyme label, the detection can be accomplished by colorimetric methods which employ a substrate for the enzyme. Detection can also be accomplished by visual comparison of the extent of enzymatic reaction of a substrate in comparison with similarly prepared standards.

*In situ* detection can be accomplished on a specimen from a patient, by providing the labeled antibody, or the unlabelled antibody plus a labeled binding partner to such a specimen. Through the use of such a procedure, it is possible to determine not only the presence of the antigen but also its distribution in the examined tissue. Those of ordinary skill will readily perceive that any of a wide variety of histological methods (such as staining procedures) can be modified in order to achieve such *in situ* detection. Such methods include, for example, immunohistochemical staining procedures. In an aspect, an avidinbiotin immunoperoxidase staining system can be used, and a kit utilizing this system is also contemplated, although the methods of the present invention can utilize any suitable staining procedures known in the art.

Any suitable detection system can be used in accordance with the methods of the present invention. Such detection systems are widely used in immunofluorescence applications, and can be imaged using techniques including, but not limited to, flow cytometry, microscopy, Western blotting, and ELISAs. Suitable detection systems can employ conjugates of secondary antibodies, conjugates of colloidal gold, or conjugates of secondary proteins, in order to amplify the signal from a primary protein (in the context of the present invention, the primary protein signal being amplified is bound a sGMF-B antibody, which can or cannot be labeled, for example with a protein such as biotin), which is in turn being used to detect a specific target (in the context of the present invention, the target is a *GMF-B* expression product).

Suitable secondary conjugates for use in the methods of the present invention can include, but are not limited to, enzyme conjugates of a secondary antibody and an enzyme such as horseradish peroxidase or alkaline phosphatase; enzyme conjugates of avidin or streptavidin and an enzyme such as horseradish peroxidase or alkaline phosphatase; enzyme conjugates of protein A or protein G and an enzyme such as horseradish peroxidase or alkaline phosphatase; conjugates of colloidal gold and a secondary antibody; conjugates of colloidal gold and avidin or streptavidin; conjugates of magnetic particles and a secondary antibody; and conjugates of secondary antibodies and labels such as fluorescent dyes and biotin. The present invention is not limited to any particular detection systems, and it is considered within the ability of the person of ordinary skill in the art to utilize these or other detection systems in accordance with the present invention. These secondary conjugates (also referred to as labels in the context of the present invention) are useful for visualizing antigen-antibody complexes.

The antibody or antibody fragment can also be adapted for utilization in an immunometric assay, also known as a "two-site" or "sandwich" assay. In a typical immunometric assay, a quantity of unlabelled antibody (or fragment of antibody), is bound to a solid support that is insoluble in the fluid being tested and a quantity of detectably labeled soluble antibody is added to permit detection and/or quantitation of the ternary complex formed between solid-phase antibody, antigen, and labeled antibody.

### 3. Antibodies and Antibody Fragments

Disclosed are antibodies and antibody fragments capable of binding preferential for GMF-B antigens. Antibodies or antibody fragments include those that are specific or preferentially selective for at least one GMF-B form. In certain embodiments, the antibodies and fragments thereof can be used to detect a soluble and/or secreted form of a GMF-B protein. A soluble GMF-B protein has a molecular weight of about 17 kDa, as measured by gradient polyacrylamide gel electrophoresis.
In certain embodiments, the antibodies and antibody fragments are capable of binding to a soluble form of GMF-B (sGMF-B) with a specific affinity of between 10-⁸ M and 10-¹¹ M; an antibody or antibody fragment capable of binding to a sGMF-B in a cell; an antibody or antibody fragment capable of selectively reducing the activity of a soluble GMF-B in a cell; and/or an antibody or antibody fragment capable of preferentially binding to a sGMF-B.

An antibody or antibody fragment can be any antibody or antibody fragment and, without limitation, can be a monoclonal antibody, a chimeric antibody, a humanized antibody, or an antibody conjugate.

In an aspect, an antibody or antibody fragment can be any gamma globulin protein found in blood or other bodily fluids of vertebrates, and used by the host immune system to identify and neutralize foreign objects, such as bacteria and viruses. In one aspect, the antibody or antibody fragment can be selected from an antibody, a monoclonal antibody, a chimeric antibody, a humanized antibody, or an antibody conjugate. In an aspect, an antibody or antibody fragment can be any type of immunoglobulin protein, such as IgA, IgD, IgE, IgG or IgM.

In one aspect, an antibody or antibody fragment is capable of reducing the activity of GMF-B in at least one form, including a soluble form. In another aspect, an antibody or antibody fragment is capable of reducing the activity of GMF-B in a secreted form. GMF-B activity is determined by measuring the poly(rC) binding of a sample. In an aspect, the poly(rC)-binding assay is carried out using a gel-shift assay as described in Ausubel FM, (1994). Current Protocols in Molecular Biology. Chichester: John Wiley and Sons.

In another aspect of the present invention, an antibody or antibody fragment is capable of preferentially binding to a secreted form of GMF-B protein. In one aspect of the present invention, an antibody or antibody fragment is capable of preferentially binding to a soluble form of GMF-B protein. In another aspect of the present invention, an antibody or antibody fragment is capable of binding to a secreted and soluble form or forms of GMF-B protein. In such aspects, such preferential binding GMF-B can be relative to any other protein. In a particular aspect, such preferential binding to GMF-B is relative to GMF-B that is nuclear bound or associated. In another particular aspect, such preferential binding to GMF-B is relative to GMF-B that is nuclear bound or associated. In another aspect of the present invention, antibodies or antibody fragments can be used to detect a secreted form of GMF-B. In another aspect of the present invention, antibodies or antibody fragments can be used to detect a soluble and secreted form or forms of GMF-B.

In an aspect of the present invention, preferential binding is relative to background. In another aspect, the preferential binding is at least 2-fold, 3-fold, 4-fold, 5-fold, 101-fold, 100-fold, 1,000-fold, or 1,000,000-fold. In another aspect, an antibody of the present invention preferentially binds a soluble form of GMF-B compared to a nuclear form of GMF-B. In a particular aspect, an antibody of the present invention preferentially binds a soluble form of GMF-B compared to a nuclear form of GMF-B, or the reverse, in another aspect. A binding of the antibody can be measured in any way, and a preferred methodology is a gel-shift assay, set forth in Ausubel.

In an aspect, an antibody or antibody fragment binds GMF-B or a particular form of GMF-B such as a soluble form, a secreted form, and/or a nuclear bound form with a specific affinity of greater than 10-⁷M, 10-⁸M, 10-⁹M, 10-¹⁰M, or 10-¹¹M, between 10-⁸M-10-¹¹M, 10-⁹M-10-¹⁰M, and 10-¹⁰M-10-¹¹M. In a preferred aspect, specific activity is measured using a competitive binding assay as set forth in Ausubel.

Antibodies and antibody fragments can optionally be immobilized on a solid phase, detectably labeled, or conjugated to a cytotoxic radionuclide, a cytotoxic drug, or a cytotoxic protein and the like.

Antibodies and antibody fragments include those capable of binding to the GMF-B epitopes comprising or consisting of SEQ ID NO: 15, SEQ ID NO: 16 or SEQ ID NO: 17 or fragments of these amino acids. In another aspect, antibodies or antibody fragments can preferentially be used to detect the GMF-B epitopes comprising or consisting of SEQ ID NO: 15, SEQ ID NO: 16 or SEQ ID NO: 17 or fragments of these amino acid sequences. Also disclosed are antibodies and antibody fragments specific to GMF-B expression products that contain antigen binding sites that are substantially homologous to these, or that result in substantially similar binding properties. Such antibodies or fragments thereof can be capable of binding to epitopes that are 95%, 90%, 85%, or 80% identical to one or more of the GMF-B epitopes comprising or consisting of SEQ ID NO: 15, SEQ ID NO: 16 or SEQ ID NO: 17 or fragments of these amino acids.

Also disclosed is an antibody or an antibody fragment with preferential binding for a GMF-B antigen, including at least one of the heavy chain CDR antigen binding site amino acid sequences CDR1, CDR2, and CDR3 (SEQ ID NOs: 2, 3, and 4) as set forth in Figure 1, and at least one of the light chain CDR antigen binding site amino acid sequences CDR1, CDR2 and CDR3 (SEQ ID NOs. : 6, 7, and 8) as set forth in Figure 2. Also disclosed is an antibody with preferential binding for a GMF-B antigen, comprising one or more of the heavy chain CDR antigen binding site amino acid sequences set forth in Figure 1, and one or more of the light chain CDR antigen binding site amino acid sequences set forth in Figure 2.

Also disclosed are GMF-B antibodies or antibody fragments having antigen binding sites with one or more of CDR1, CDR2, and CDR3, from both heavy and light chains, as described in Figures 1 and 2. An antibody or antibody fragment may include any single CDR shown in Figures 1 and 2, alone or in combination. By way of example, an antibody or antibody fragment may include CDR1 and CDR2 from both heavy and light chains of Figures 1 and 2 (SEQ ID NOs.: 2, 3, 6, and 7, respectively). An antibody or antibody fragment may include CDR1, CDR2, CDR3 from both heavy and light chains of Figures 1 and 2 (SEQ ID NOs.: 2, 3, 4, 6, 7, and 8, respectively). An antibody or antibody fragment may include the full heavy and light chain sequences illustrated in Figures 1 and 2 (SEQ ID NOs.: 1, 21 and 5, 39).

Also disclosed are antibodies and antibody fragments specific to GMF-B expression products that contain antigen binding sites that are substantially homologous to these, or that result in substantially similar binding properties. Such antibodies or fragments thereof comprises sequences 95%, 90%, 85%, or 80% identical to one or more of the CDR1, CDR2, or CDR3 heavy or light chain from Figures 1 and 2. Also disclosed are new hybridoma lines, and the monoclonal antibody molecules that they secrete, which are specific to GMF-B antigen expressed by normal or cancer cells. Also disclosed are chimeric, such as humanized antibodies, and antibody fragments and other modified antibodies and antibody fragments.

In addition to the specific amino acid sequences of the antigen binding sites of the heavy and light chains set forth in Figures 1 and 2, also disclosed are antibodies and antibody fragments that have preferential binding to GMF-B antigens but which have FWR and/or CDR antigen binding site amino acid sequences that are not identical to those set forth in Figures 1 and 2. Such antibodies and antibody fragments are preferred if they are specific or preferentially selective for the sGMF-B antigen, preferably at least 85% or more as specific, more preferably at least 90% or more as specific, and most preferably at least 95% or more as specific for the sGMF-B antigen as the Alper-sGMF-B mAb or antibody fragment therefor. According to a preferred aspect, a variant of an antibody or antibody fragment of the present invention can be as specific for the GMF-B antigen as a non-variant antibody or antibody fragment of the present invention, or can be more specific.

Antibodies and antibody fragments that are specific to sGMF-B but which have FWR and/or CDR antigen binding site amino acid sequences that are not identical to those set forth in Figures 1 and 2 can possess the same or different specificity determining regions (SDRs) as the FWRs and/or CDRs of Figures 1 and 2 are included (set forth in bold, underlined text in these figures).

Modifications to the amino acid sequences of the antigen binding sites CDR1, CDR2, and CDR3 set forth in Figure 1 (heavy chain) and Figure 2 (light chain) can occur in either or both of the FWR and CDR sequences. Variations in antibodies or antibody fragments can occur where they have substantially homologous amino acid sequences, antibodies having substantially similar binding properties, or both.

Humanized variants of the antibodies or antibody fragments can contain a reduced murine content, and potentially, reduced immunogenicity, when compared to murine antibodies, such as Alper-sGMF-B mAb, or antibody fragments thereof.
Humanized variants include those that retain a binding affinity that is substantially similar to that of the original antibody or antibody fragment. Also disclosed are CDR variants of humanized GMF-B antibodies or antibody fragments in which 1, 2, 3, 4, 5, or 6 (three heavy chain and three light chain) CDRs are humanized. Also disclosed are SDR variants of humanized GMF-B antibodies and antibody fragments in which only Specificity Determining Residues (SDRs) of at least one CDR from the GMF-B antibodies and antibody fragments are present in the humanized antibodies. The SDRs are selected from Table 1 or Table 2.

**Table 1. Specificity-Determining Residues in Alper-sGMF-B mAb Heavy Chain (SEQ ID NO. 1 and 21). Residues 98-115 disclosed as SEQ ID NO: 18.**

| **Position** | **Residue** |
|---|---|
| 4 | Q |
| 8 | P |
| 10 | L |
| 11 | V |
| 19 | M |
| 30 | S |
| 32 | V |
| 37 | K |
| 39 | K |
| 47 | I |
| 49 | Y |
| 53 | Y |
| 54 | N |
| 55 | E |
| 58 | K |
| 60 | N |
| 61 | E |
| 64 | K |
| 66 | K |
| 67 | A |
| 69 | L |
| 71 | S |
| 73 | K |
| 75 | S |
| 84 | S |
| 86 | T |
| 88 | E |
| 90 | S |
| 98 | S |
| 99 | T |
| 100 | M |
| 101 | I |
| 102 | T |
| 103 | T |
| 104 | G |
| 105 | F |
| 106 | A |
| 107 | Y |
| 108 | W |
| 109 | G |
| 110 | Q |
| 111 | G |
| 112 | T |
| 113 | T |
| 114 | V |
| 115 | T |

**Table 2. Specificity-Determining Residues in Alper-sGMF-B mAb Light Chain (SEQ ID NO. 5 and 39). Residues 95-106 disclosed as SEQ ID NO: 19.**

| **Position** | **Residue** |
|---|---|
| 3 | L |
| 15 | L |
| 17 | G |
| 18 | K |
| 24 | K |
| 30 | N |
| 31 | K |
| 33 | I |
| 34 | A |
| 38 | H |
| 42 | E |
| 43 | G |
| 45 | R |
| 49 | H |
| 50 | Y |
| 51 | T |
| 52 | T |
| 55 | Q |
| 56 | P |
| 58 | I |
| 69 | R |
| 71 | Y |
| 72 | S |
| 74 | S |
| 76 | T |
| 77 | N |
| 79 | E |
| 89 | L |
| 95 | _{W} |
| 96 | T |
| 97 | F |
| 98 | G |
| 99 | G |
| 100 | G |
| 101 | T |
| 102 | K |
| 103 | L |
| 104 | E |
| 105 | I |
| 106 | K |

CDR variants can be formed by replacing at least one CDR of a humanized GMF-B antibody and antibody fragments with a corresponding CDR from a human antibody. CDR variants in which one, two, three, four, five, or six CDRs are replaced by a corresponding CDR from a human antibody and retain biological activity that is substantially similar to the binding affinity of the parental GMF-B mAb. CDR variants can have a binding affinity that is 25% more than the binding affinity of the parental sGMF-B antibody or antibody fragment, more preferably is more than 50%, most preferably at least 75% or 90%.

CDR variants can have altered immunogenicity when compared to GMF-B antibodies and antibody fragments can be formed by grafting all six (three heavy chain and three light chain) CDRs from the GMF-B antibodies and antibody fragments onto the variable light (VL) and variable heavy (VH) frameworks of human antibodies and antibody fragments. However, less than all six of the CDRs of the GMF-B antibodies and antibody fragments can be present, while still permitting an antibody to retain activity. Residues that are directly involved in antigen contact, such as Specificity Determining Residues (SDRs), can be refined. SDR variants are formed by replacing at least one SDR of the GMF-B antibody or antibody fragment with a residue at a corresponding position from a human antibody. It should be noted that not all CDRs must include SDRs.

In a preferred aspect, the variants of the present antibodies and antibody fragments include a combination of CDR and/or SDR substitutions to generate variants having reduced immunogenicity in humans and a binding affinity that is substantially similar to that of the parental antibody or antibody fragment to sGMF-B.

In addition to variants specifically described herein, other "substantially homologous" modified immunoglobulins can be readily designed and manufactured using various recombinant DNA techniques. For example, the framework regions (FWRs) can be varied at the primary structure level. Moreover, a variety of different human framework regions can be used singly or in combination as a basis for the variant. In general, modifications of the genes can be readily accomplished by a variety of techniques, such as site-directed mutagenesis and random mutagenesis.

Alternatively, polypeptide fragments comprising only a portion of the primary antibody structure can be produced where the fragment substantially retains the immunoreactivity properties of the variant. Such polypeptide fragments include fragments produced by proteolytic cleavage of intact antibodies or fragments produced by inserting stop codons at the desired locations nucleotide sequence using site-directed mutagenesis. Single chain antibodies and fusion proteins which include at least an immunoreactivity fragment of the variant are also included.

Antibodies and antibody fragments can either be labeled or unlabeled. Unlabeled antibodies can be used in combination with other labeled antibodies (second antibodies) that are reactive with the humanized antibody, such as antibodies specific for human immunoglobulin constant regions. Alternatively, the antibodies can be directly labeled. A wide variety of labels can be employed, such as radionuclides, fluors, enzymes, enzyme substrates, enzyme cofactors, enzyme inhibitors, ligands (particularly haptens), etc. Numerous types of immunoassays are available.

Once candidate drugs have been developed based on the GMF-B antigens, the sGMF-B antigens and GMF-B antibodies and antibody fragments can be used to aid in screening the various drug candidates, in order to identify those drug candidates that exhibit a desired level of specificity for diseased cells presenting sGMF-B expression products.

The following examples are non-limiting illustrative examples.

### Example 1: Breast cancer patients have higher sGMF-B levels than control groups.

Plasma samples are obtained from control and breast cancer patient groups and are diluted with PBS at a ratio of 1: 100. Plasma sGMF-B levels are measured with an enzyme-linked immunosorbent enzyme assay. The polysorp ELISA plates (Nalgene NUNC® International, Rochester, NY) are coated with 100 µl/well of diluted plasma and incubated at 4°C overnight. The blood plasma samples are analyzed in a blinded fashion. Wells are washed with PBS and incubated at room temperature for one hour with blocking buffer (5% BSA in PBS). After washing with PBS, the primary antibody, anti-sGMF-B mAb (clone name: Alper-GMF-B) is added in dilution buffer (45 µg/ml) (PBS buffer, 1% BSA, 0.01% Tween-20). The wells are washed with PBS/0.03% Tween-20 and incubated at room temperature for one hour with 100 µl/well secondary antibody (HRP-Donkey anti-mouse IgG, Jackson ImmunoResearch, West Grove, PA) diluted 1 :3000. After washing the wells, 100 µl Immunopure TMB substrate solution (Pierce, Rockford, IL) is added. Color reaction is stopped by the addition of 100 µl/well IN H₂SO₄ and the analysis is performed with an ELISA Reader. The figure represents optical density (OD) values of plasma readings for sGMF-B levels. *See* Figure 3.

### Example 2: GMF-B expression is indicative of a bad overall prognosis.

A 700 breast cancer patient cohort is used for staining. Microtome sections are deparaffinized and incubated with Anti-sGMF-B mAb (clone name: Alper-GMFB) following general immunohistochemistry (IHC) procedures. Diagnostic staining is performed via standard pathological procedures using Her2, estrogen receptor, and progesterone receptor antibodies. Results show that the presence of glia maturation factor beta (GMFB) protein expression is indicative for bad prognosis and lymph node metastasis in triple negative (ER, PR and HER2) primary (mostly stage IIb) breast cancer tissues. Stage IIb is designated when the tumor is either larger than 2 centimeter but not larger than 5 centimeters and has spread to the auxiliary lymph nodes, or larger than 5 centimeters but has not spread to the auxiliary lymph nodes. Absence of GMFB expression is indicative of good prognosis and no lymph node metastasis in triple negative primary breast cancer patients, showing negative staining pattern using AB-GMFbeta IHC kit with Alper GMFbeta moab.

Presence of glia maturation factor beta expression is indicative of bad prognosis in overall survival in patients (n=700 breast cancer patient cohort) with ER and PR negative status. Absence of glia maturation factor beta expression is indicative of good prognosis in overall survival in patients (n=700 breast cancer patient cohort) with ER and PR positive status.

One hundred primary breast cancer tissues are stained with AB-GMFbeta IHC kit, and 36% of these tissues showed positive staining (2+ and 3+) for GMFbeta in triple negative (ER, PR, HER2) and stage II and III breast tissue sections.

Fifty-eight percent of both metastatic breast cancer tissue sections and their corresponding lymph node tissue sections show positive staining (2+ and 3+) for GMFbeta in stage IIb breast cancer patients when stained with AB-GMFbeta IHC kit with Alper-GMFbeta moab. Twenty-five percent of metastatic breast cancer patients do not show lymph node staining for GMFbeta, even though their metastatic breast tissues showed positive staining for GMFbeta.

Slides are prepared according to the following protocol. Incomplete removal of paraffin can cause poor staining of the section. Accordingly, prior to staining, tissue sections are deparaffinized and rehydrated as follows: immerse slides in xylene and incubate for 2 x 15 minutes; immerse slides in xylene: ethanol (1:) for 5 minutes; immerse slides in 100% ethanol for 5 minutes, and follow with 95%, 75% and 50% ethanol for 3 minutes each; rinse slides with reagent-quality water for 5 minutes and keep in water until ready to perform antigen retrieval.

Following deparaffinization and rehydration, antigen is retrieved using heat induced antigen retrieval (HIAR) as follows: fill plastic Coplin Jar/container with Retrieval Buffer; place the Coplin jar/container in steamer; turn on steamer and preheat to 90-100 °C; carefully put slides into the Coplin jar/container and steam for 40 min (95-100 °C); turn off the steamer, remove the Coplin jar/container to room temperature and allow the slides to cool for 20 min; rinse slides with Wash Buffer for 3 x 3 minutes and begin staining procedure.

The retrieved antigen is then stained for immunohistochemical analysis as follows: tap off excess buffer. Apply enough Peroxidase Blocking Buffer (3% hydrogen peroxide) to cover specimen, and incubate for 5 minutes; rinse sections with Washing Buffer for 3 x 3 minutes; tap off excess buffer. Apply enough Blocking Reagent to cover specimen and incubate for 5 minutes; rinse sections with washing buffer for 3 x 3 minutes; tap off excess buffer. Apply enough GMFB antibody (1 :50 dilution) to cover specimen, and incubate for 1 hour. Sections are rinsed with washing buffer for 3 x 3 minutes; tap off excess buffer. Apply enough Mach3 probe to cover specimen, and incubate for 15 minutes; sections are rinsed with washing buffer for 3 x 3 minutes; tap off excess buffer. Apply enough Mach3 polymers to cover specimen, and incubate for 15 minutes; rinse sections with Washing Buffer for 3 x 3 minutes; tap off excess washing buffer; apply enough DAB substrate solution to cover specimen, and incubate until desired stain intensity develops; rinse sections in tap water for 3 minutes; immerse slides in hematoxylin solution, incubate 30 sec to 5 minutes; rinse to clear with tap water and follow by dehydration; immerse slides in 70%, 80%, 95%, 100% ethanol for 2 minutes each, and follow in xylene for 2 x 2 minutes; dry and mount slides.

The positive staining both cytoplasmic and strongly nuclear in GMF-B expression was found to be significantly enhanced in invasive ductal breast cancer cells than that in normal cells, normal conditions and was positively correlated with stage, TNM classification. *See* Figure 4.

### Example 3: GMFB expression is significantly associated with increased survival of ER or PR negative breast cancer patients.

A total of 714 breast tumor and control samples are obtained from Yale School of Medicine, Department of Pathology, Tissue Microarray and Archiving, YTMA. Of these samples, 630 are from female breast cancer patients. These are samples utilized for assessment of GMF-B expression using anti-GMFB mAb (clone name: Alper-GMF-B). Available patient characteristics are examined for any association with overall survival time using the long rank test. Overall survival was measured as the number of months from diagnosis to death or last contact. Patients without dates of death were censored on their date of last contact. Nuclear grade was omitted from multivariable analyses due to the number of samples missing this information. Kaplan-Meier plots present the estimated survival for different groups.

Figure 4 presents the overall survival curve based on GMF-B status, where 2+ and 3+ are GMFB+ and less expression is GMFB(-). All patients have died or have last follow-up by 500 months except for one patient who died after 660 months. As shown in Figure 4, GMFB expression is significantly associated (p < 0.01) with increased survival of ER or PR negative breast cancer patients.

What has been described and illustrated herein are exemplary embodiments of the invention along with some of its variations. The terms, descriptions and figures used herein are set forth by way of illustration only and are not meant as limitations. Those skilled in the art will recognize that many variations are possible within the scope of the invention, which is intended to be defined by the following claims, in which all terms are meant in their broadest reasonable sense unless otherwise indicated therein.

## Claims

1. A method of predicting disease progression comprising:
determining the expression of glia maturation factor beta in an obtained sample
of breast tissue, wherein expression of glia maturation factor beta is indicative of lymph node metastatis.

2. A method of predicting disease progression comprising:
determining the expression of glia maturation factor beta in an obtained sample of breast tissue, wherein expression of glia maturation factor beta is indicative of an untreated or treated prognosis that is reduced compared to an absence of said expression.

3. A method of predicting disease progression comprising:
determining the expression of glia maturation factor beta in an obtained sample of breast tissue, wherein non-expression of glia maturation factor beta is indicative of an enhanced untreated or treated prognosis compared to the presence of said expression.

4. A method of any of claims 1 to 3, wherein said tissue is obtained from a stage IIB breast cancer.

5. A method of any of claims 1 to 3, wherein said determining is obtained with an antibody to glia maturation factor beta.

6. A method of claim 5, wherein said antibody shows cytoplasmic staining.

7. A method of claim 5, wherein said antibody shows nuclear staining.

8. A method of claim 6, wherein said antibody further shows nuclear staining.

9. A method according to any of claims 1 to 3, wherein said expression is associated with selecting a clinical approach.

10. A method according to any of claims 1 to 3, wherein said breast cancer is obtained from a tumor larger than 5 cm.

11. A method according to claim 1, wherein said lymph node metastatis is confirmed by biopsy.

12. A method according to any preceding claim wherein the breast tissue is estrogen receptor negative, progesterone receptor negative and does not over-express human epidermal growth factor 2 receptor protein.

13. An immunoassay method for detecting a GMB-B antigen which binds to an antibody or antibody fragment specific for a GMF-B antigen, comprising one or more of the heavy-chain CDR antigen binding site sequences selected from the group consisting of SEQ ID NOs: 2, 3, and 4, and one or more of the light-chain CDR antigen binding site sequences selected from the group consisting of SEQ ID NOs: 6, 7, and 8, comprising:
(a) contacting an obtained sample of breast tissue, wherein the breast tissue is estrogen receptor negative, progesterone receptor negative and does not over-express human epidermal growth factor 2 receptor protein, with an effective binding amount of said antibody or antibody fragment; and
(b) detecting said antigen by detecting the binding of the antibody to the GMF-B antigen, wherein said binding is prognostic of disease progression.

## Patentansprüche

1. Verfahren zum Vorhersagen des Krankheitsverlaufs, umfassend:
Bestimmen der Expression des Glia-Reifungsfaktors-beta in einer aus Brustgewebe erhaltenen Probe, wobei die Expression des Glia-Reifungsfaktors-beta indikativ für Lymphknotenmetastasen ist.

2. Verfahren zum Vorhersagen des Krankheitsverlaufs, umfassend:
Bestimmen der Expression des Glia-Reifungsfaktors-beta in einer aus Brustgewebe erhaltenen Probe, wobei die Expression des Glia-Reifungsfaktors-beta indikativ für eine nicht behandelte oder behandelte Prognose ist, die im Vergleich zur Abwesenheit der Expression geringer ist.

3. Verfahren zum Vorhersagen des Krankheitsverlaufs, umfassend:
Bestimmen der Expression des Glia-Reifungsfaktors-beta in einer aus Brustgewebe erhaltenen Probe, wobei die Nicht-Expression des Glia-Reifungsfaktors-beta im Vergleich zur Anwesenheit der Expression indikativ für eine bessere nicht behandelte oder behandelte Prognose ist.

4. Verfahren nach einem der Ansprüche 1 bis 3, wobei das Gewebe aus einem Brustkrebs im Stadium IIB erhalten wird.

5. Verfahren nach einem der Ansprüche 1 bis 3, wobei das Bestimmen mit einem Antikörper gegen den Glia-Reifungsfaktor-beta erhalten wird.

6. Verfahren nach Anspruch 5, wobei der Antikörper zytoplasmatische Anfärbung zeigt.

7. Verfahren nach Anspruch 5, wobei der Antikörper Kernfärbung zeigt.

8. Verfahren nach Anspruch 6, wobei der Antikörper weiter Kernfärbung zeigt.

9. Verfahren nach einem der Ansprüche 1 bis 3, wobei die Expression mit der Auswahl eines klinischen Ansatzes verbunden ist.

10. Verfahren nach einem der Ansprüche 1 bis 3, wobei der Brustkrebs aus einem Tumor erhalten wird, der größer ist als 5 cm.

11. Verfahren nach Anspruch 1, wobei die Lymphknotenmetastase durch Biopsie bestätigt wird.

12. Verfahren nach einem der vorstehenden Ansprüche, wobei das Brustgewebe negativ für den Östrogenrezeptor und negativ für den Progesteronrezeptor ist und das Rezeptorprotein des humanen epidermalen Wachstumsfaktors 2 nicht überexprimiert.

13. Immunassayverfahren zum Nachweisen eines GMB-B-Antigens, das an einen für ein GMF-B spezifischen Antikörper oder Antikörperfragment bindet, umfassend eine oder mehr der Sequenzen der CDR-Antigen-Bindungsstelle der schweren Kette, ausgewählt aus der Gruppe bestehend aus SEQ ID NO: 2, 3 und 4, und eine oder mehr der Sequenzen der CDR-Antigen-Bindungsstelle der leichten Kette, ausgewählt aus der Gruppe bestehend aus SEQ ID NO: 6, 7 und 8, umfassend:
(a) Inkontaktbringen einer aus Brustgewebe erhaltenen Probe, wobei das Brustgewebe negativ für den Östrogenrezeptor und negativ für den Progesteronrezeptor ist und das Rezeptorprotein des humanen epidermalen Wachstumsfaktors 2 nicht überexprimiert, mit einer wirksamen Bindungsmenge des Antikörpers oder des Antikörperfragments; und
(b) Nachweisen des Antigens durch Nachweis der Bindung des Antikörpers an das GMF-B-Antigen, wobei die Bindung prognostisch für den Krankheitsverlauf ist.

## Revendications

1. Procédé de prédiction d'une progression de maladie comprenant :
une détermination de l'expression du beta facteur maturation névroglie dans un échantillon obtenu de tissu mammaire, dans lequel l'expression du beta facteur maturation névroglie est indicative d'un métastase de noeud lymphoïde.

2. Procédé de prédiction d'une progression de maladie comprenant :
une détermination de l'expression du beta facteur maturation névroglie dans un échantillon obtenu de tissu mammaire, dans lequel l'expression du beta facteur maturation névroglie est indicative d'un pronostic non traité ou traité qui est réduit comparé à une absence de ladite expression.

3. Procédé de prédiction d'une progression de maladie comprenant :
une détermination de l'expression du beta facteur maturation névroglie dans un échantillon obtenu de tissu mammaire, dans lequel la non-expression du beta facteur maturation névroglie est indicative d'un pronostic non traité ou traité amélioré comparé à la présence de ladite expression.

4. Procédé selon l'une quelconque des revendications 1 à 3, dans lequel ledit tissu est obtenu à partir d'un cancer du sein de stade IIB.

5. Procédé selon l'une quelconque des revendications 1 à 3, dans lequel ladite détermination est obtenue avec un anticorps contre le beta facteur maturation névroglie.

6. Procédé selon la revendication 5, dans lequel ledit anticorps montre une coloration cytoplasmique.

7. Procédé selon la revendication 5, dans lequel ledit anticorps montre une coloration nucléaire.

8. Procédé selon la revendication 6, dans lequel ledit anticorps montre en outre une coloration nucléaire.

9. Procédé selon l'une quelconque des revendications 1 à 3, dans lequel ladite expression est associée à une sélection d'une approche clinique.

10. Procédé selon l'une quelconque des revendications 1 à 3, dans lequel ledit cancer du sein est obtenu à partir d'une tumeur de plus de 5 cm.

11. Procédé selon la revendication 1, dans lequel ledit métastase de noeud lymphoïde est confirmé par biopsie.

12. Procédé selon une revendication précédente quelconque dans lequel le tissu mammaire est à récepteurs d'oestrogènes négatifs, à récepteurs de progestérone négatifs et ne surexprime pas la protéine réceptrice du facteur 2 de croissance épidermique humaine.

13. Procédé immunoessai de détection d'un antigène GMB-B qui se lie à un anticorps ou fragment d'anticorps spécifique d'un antigène GMF-B, comprenant une ou plusieurs des séquences de site de liaison d'antigène CDR à chaîne lourde sélectionnées à partir du groupe consistant en SEQ ID NOs : 2, 3, et 4, et une ou plusieurs des séquences de site de liaison d'antigène CDR à chaîne légère sélectionnées à partir du groupe consistant en SEQ ID NOs : 6, 7, et 8, comprenant :
(a) une mise en contact d'un échantillon obtenu de tissu mammaire, dans laquelle le tissu mammaire est à récepteurs d'oestrogènes négatifs, à récepteurs de progestérone négatifs et ne surexprime pas la protéine réceptrice du facteur 2 de croissance épidermique humaine, avec une quantité de liaison effective dudit anticorps ou fragment d'anticorps ; et
(b) une détection dudit antigène en détectant la liaison de l'anticorps à l'antigène GMF-B, dans laquelle ladite liaison est un pronostic d'une progression de maladie.
